(19) Europäisches Patentamt
European Patent Office
Office européen des brevets

(11) EP 1 845 094 A1

(12) # EUROPEAN PATENT APPLICATION
published in accordance with Art. 158(3) EPC

(43) Date of publication:
**17.10.2007 Bulletin 2007/42**

(21) Application number: **06700524.9**

(22) Date of filing: **04.01.2006**

(51) Int Cl.:
***C07D 277/58*** *(2006.01)* ***A61K 31/426*** *(2006.01)*
***A61P 35/00*** *(2006.01)* ***C07D 403/04*** *(2006.01)*
***A61K 31/404*** *(2006.01)* ***C12N 15/09*** *(2006.01)*

(86) International application number:
**PCT/JP2006/300160**

(87) International publication number:
**WO 2006/073202 (13.07.2006 Gazette 2006/28)**

(84) Designated Contracting States:
**DE FR GB**

(30) Priority: **04.01.2005 JP 2005000133**

(71) Applicant: **National University Corporation Kanazawa University**
**Kanazawa-shi,**
**Ishikawa 920-1164 (JP)**

(72) Inventor: **MINAMOTO, T.,**
**Nat. Univ. Corp. Kanazawa Univ.**
**Kanazawa-shi,**
**Ishikawa 9201164 (JP)**

(74) Representative: **Vossius & Partner**
**Siebertstrasse 4**
**81675 München (DE)**

(54) **METHOD OF TUMOR SUPPRESSION AND EVALUATION OF ANTICANCER AGENT BASED ON GSK3 BETA INHIBITORY EFFECT**

(57) The present invention provides a novel means for cancer treatment and cancer diagnosis by elucidating that glycogen synthase kinase 3β (GSK3β) is involved in the survival and/or the proliferation of cancer cells. Specifically, the present invention relates to: a method for suppressing the survival and/or the proliferation of cancer cells through inhibition of GSK3β expression or inhibition of the activity of this enzyme (GSK3β) activated via phosphorylation of GSK3β tyrosine residue at position 216; and development of novel anticancer agents and a method for evaluating anticancer agents based on the inhibitory effect.

Fig. 7

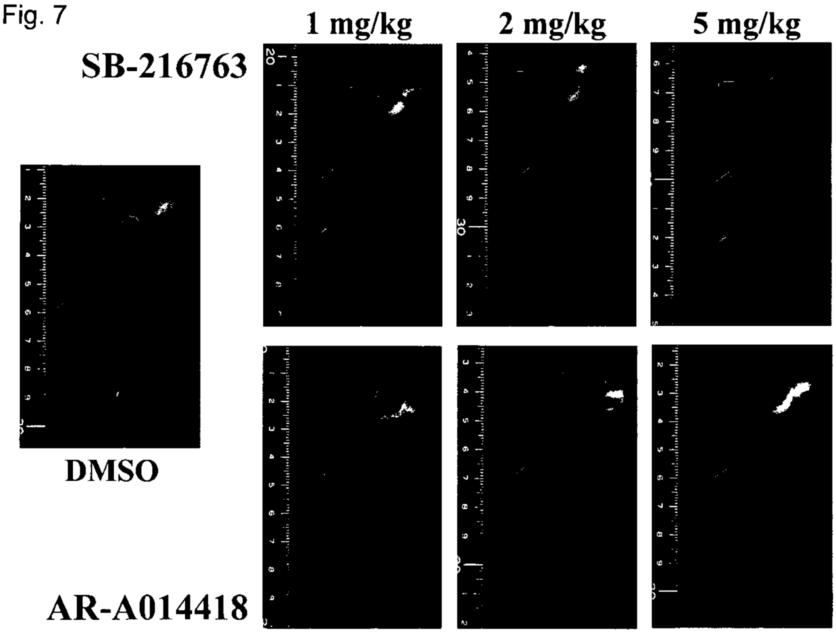


EP 1 845 094 A1

## Description

### Technical Field

The present invention relates to a method for suppressing the survival and/or the proliferation of cancer cells through inhibition of the activity of glycogen synthase kinase 3β (GSK3β) in which tyrosine residue at position 216 is phosphorylated or through inhibition of the phosphorylation of GSK3β tyrosine residue at position 216 and development of a novel anticancer agent and a method for evaluating an anticancer agent based on the above inhibitory effect.

### Background Art

In recent years, concerning the molecular mechanism of colorectal cancer development and progression, attention has been drawn to the activation of β-catenin via its stabilization as a potent novel oncogenic signaling, together with the oncogenic effect of the ras oncogene product (see Jass JR, Whitehall VLJ, Young J, Leggett BA, Emerging concepts in colorectal neoplasia, Gastroenterology 2002, 123(3): 862-76; and Wong NA, Pignatelli M, β-catenin-a linchpin in colorectal carcinogenesis, Am J Pathol 2002, 160(2): 389-401). β-catenin is a central factor that participates in the Wnt signaling pathway involving cell proliferation. The oncogenic effect of β-catenin has been supported by many studies using *in vitro* experimental systems and transgenic animal models (see Polakis P, The oncogenic activation of beta-catenin, Curr Opin Genet Dev 1999, 9(1): 15-21).

The present inventors have recently discovered the presence of two types of β-catenin activation patterns in human colorectal cancer based on differences in β-catenin accumulation patterns in tumor cell nuclei (see Ougolkov AV, Yamashita K, Mai M, Minamoto T, Oncogenic β-catenin and MMP-7 (matrilysin) cosegregate in late-stage clinical colon cancer, Gastroenterology 2002, 122(1): 60-71). In particular, whereas β-catenin nuclear accumulation (NA), that is, activation, is observed throughout the tumor (NAd) in 40%-50% of colorectal cancer cases, localized accumulation (activation) is detected at the invasion front of the tumor (NAinv) in 10%-15% of the cases. The NAinv pattern of β-catenin activation is significantly correlated with advancement in the disease stage and the postoperative recurrence of colorectal cancer. The results suggest that the oncogenic activation pattern of β-catenin in colorectal cancer can be a reliable factor for predicting tumor metastasis, tumor recurrence, and postoperative survival rate of patients (see Ougolkov AV, Yamashita K, Mai M, Minamoto T, Oncogenic β-catenin and MMP-7 (matrilysin) cosegregate in late-stage clinical colon cancer, Gastroenterology 2002, 122(1), 60-71; and Zhang B, Ougolkov A, Yamashita K, Takahashi Y, Mai M, Minamoto T, β-catenin and ras oncogenes detect most human colorectal cancers, Clin Cancer Res 2003, 9(8): 3073-9).

Glycogen synthase kinase 3β (hereinafter, referred to as "GSK3β") is a member of a control factor group involving the Wnt/β-catenin-signaling pathway (see Wong NA, Pignatelli M, β-catenin-a linchpin in colorectal carcinogenesis, Am J Pathol 2002, 160(2), 389-401; and Polakis P, The oncogenic activation of beta-catenin, Curr Opin Genet Dev 1999, 9(1): 15-21). GSK3β phosphorylates β-catenin in cells under physiological conditions and induces the product to a ubiquitin degradation pathway composed of the APC tumor suppressor protein, axin (both are GSK3β substrates), and the like. This suggests that a theoretical contact point between GSK3β activity and colorectal cancer is that inactivation of GSK3β accompanying the phosphorylation of GSK3β serine residue at position 9 causes β-catenin (target substrate) to avoid the ubiquitin degradation pathway, resulting in the activation of the oncogenic signaling.

GSK3β is multifunctional serine/threonine kinase (phosphorylation enzyme) that mediates various types of intracellular signaling. GSK3β controls wide-ranging cell regulation processes including energy metabolism, transcriptional regulation, cell proliferation and/or survival, and the like (see Manoukian AS, Woodgett JR, Role of glycogen synthase kinase-3 in cancer: regulation by Wnts and other signaling pathways, Adv Cancer Res 2002, 84: 203-29; Doble BW, Woodgett JR, GSK-3: tricks of the trade for a multi-tasking kinase, J Cell Sci 2003, 116 (Pt 7): 1175-86; Harwood AJ, Regulation of GSK-3, a cellular multiprocessor, Cell 2001, 105(7): 821-4; and Kim L, Harwood A, Kimmel AR, Receptor-dependent and tyrosine phosphatase-mediated inhibition of GSK3 regulates cell fate choice, Dev Cell 2002, 3(4): 523-32). The control of the Wnt/β-catenin signaling is only one of various functions of the enzyme. Unlike many protein kinases (phosphorylation enzymes) that mediate intracellular signaling, active form GSK3β exists in normal cells. The activity of GSK3β is regulated in a suppressive manner by various forms of stimulation. GSK3β substrates include various oncgenic transcription factors such as c-Jun and c-Myc (see Morton S, Davis RJ, McLaren A, Cohen P, A reinvestigation of the multiple phosphorylation of the transcription factor c-Jun. EMBO J 2003, 22(15): 3876-86; and Gregory MA, Qi Y, Hann SR, Phosphorylation by glycogen synthase kinase-3 controls c-myc proteolysis and subnuclear localization, J Biol Chem 2003, 278(51): 51606-12), β-catenin, and a proto-oncogene product such as Gli protein (see Price MA, Kalderon D, Proteolysis of the Hedgehog signaling effector Cubitus interruptus requires phosphorylation by glycogen synthase kinase 3 and casein kinase 1, Cell 2002, 108(6): 823-35; and Jia J, Amanai K, Wang G, Tang J, Wang B, Jiang J, Shaggy/GSK3 antagonizes Hedgehog signalling by regulating cubitus interruptus, Nature 2002, 416 (6880): 548-52). GSK3β interferes with cellular neoplastic transformation and tumorigenesis through inactivation of these substrates. Moreover, GSK3β also plays an essential role in cell survival via the NF-κB cell survival signaling (see

Hoeflich KP, Luo J, Rubie EA, Tsao MS, Jin O, Woodgett JR, Requirement for glycogen synthase kinase-3β in cell survival and NF-κB activation, Nature 2000, 406(6791): 86-90; and Schwabe RF, Brenner DA. Role of glycogen synthase kinase-3 in TNF-α-induced NF-κB activation and apoptosis in hepatocytes, Am J Physiol Gastrointest Liver Physiol 2002, 283(1): G204-11). There is also a patent application reporting that the GSK3β gene has been used as an abnormal cell proliferation marker based on such GSK3β functions (see JP Patent Publication (Kohyo) No. 2004-528837 A).

It is currently known that GSK3β is also involved in the onset of non-insulin-dependent diabetes mellitus [NIDDM] (Type 2 diabetes) and neurodegenerative diseases including Alzheimer's disease (see Cohen P, Goedert M, GSK3 inhibitors: development and therapeutic potential, Nat Rev Drug Discov 2004, 3(6): 479-87). Accordingly, the application of a GSK3β inhibitor to treatment of neurodegenerative diseases, Type 2 diabetes, cancer, and the like has also been the subject of patent applications (see JP Patent Publication (Kohyo) No. 2004-504838 A and JP Patent Publication (Kohyo) No. 2004-507545 A). Both patents merely involve predicting possible applications of such compound for the aforementioned diseases based on the effect of specific compounds to inhibit GSK3β and known findings. However, actual applications thereof have never been demonstrated, particularly concerning cancer.

As described above, GSK3β is a negative control factor of Wnt/β-catenin signaling. Thus, it is important to elucidate whether abnormalities in a GSK3β-mediated signaling-regulating function are involved in a β-catenin signaling activation pattern in cancer. Meanwhile, it has been reported that GSK3β has an effect of maintaining cell survival and/or cell proliferation mediated by the NF-κB pathway as a result of the analysis of the enzyme gene-knockout animals (see Price MA, Kalderon D, Proteolysis of the Hedgehog signaling effector Cubitus interruptus requires phosphorylation by glycogen synthase kinase 3 and casein kinase 1, Cell 2002, 108(6): 823-35; and Jia J, Amanai K, Wang G, Tang J, Wang B, Jiang J, Shaggy/GSK3 antagonizes Hedgehog signalling by regulating cubitus interruptus, Nature 2002, 416 (6880): 548-52). Furthermore, in the case of a prostate cancer-derived cell line, inhibition of GSK3β has also been reported to enhance TRAIL (tumor necrosis factor-related apoptosis-inducing ligand) sensitivity or to suppress cell proliferation (see Liao X, Zhang L, Thrasher JB, Du J, Li B, Glycogen synthase kinase-3β suppression eliminates tumor necrosis factor-related apoptosis-inducing ligand resistance in prostate cancer, Mol Cancer Ther 2003, 2(11): 1215-1222; Wang L, Lin H-K, Hu Y-C, Xie S, Yang L, Chang C, Suppression of androgen receptor-mediated transactivation and cell growth by the glycogen synthase kinase 3β in prostate cells, J Biol Chem 2004, 279(31): 32444-32452; and Mazor M, Kawano Y, Zhu H, Waxman J, Kypta RM, Inhibition of glycogen synthase kinase-3 represses androgen receptor activity and prostate cancer cell, Oncogene 2004, 23(47): 7882-7892). As described above, such conflicting effects on fundamental intracellular signaling pathways involved in cell survival and/or proliferation and cancer development have been proposed. It is thus assumed that GSK3β may have influence on cancer cells via its own molecular mechanism, which differs from that involved in Wnt signaling. However, based on the hypothesis that GSK3β acts to suppress cellular neoplastic transformation and tumorigenesis, a GSK3β inhibitor is predicted to have potential carcinogenicity. Therefore, there have been few reports concerning the involvement of GSK3β in cancer development and progression. Such involvement has little been examined in detail.

## Disclosure of the Invention

An object of the present invention is to provide a novel means for cancer treatment and diagnosis through elucidation of the involvement of glycogen synthase kinase 3β (GSK3β) in cancer development and progression.

To examine the above object, the present inventors have conducted comparative analysis concerning GSK3β expression and the degree of enzyme activation (determined via analysis of the phosphorylation of serine residue at position 9) in colon cancer cell lines and the non-cancer tissue and cancer tissue of the individual colorectal cancer patients. The present inventors have conducted modification of the transcriptional level of GSK3β expression and pharmacological modification of GSK3β activity, and thus they have studied the influence of the enzyme (GSK3β) on colorectal cancer cell proliferation.

Compared with non-cancer mucosa and cells (HEK293), GSK3β expression and the expression of an active form phospho-GSK3$\beta^{Tyr216}$ fraction having phosphorylated tyrosine residue at position 216 were found to be increased in most colorectal cancer tissues and colon cancer cell lines. On the other hand, almost no expression of an inactive form phospho-GSK3$\beta^{Ser9}$ fraction having phosphorylated serine residue at position 9 was observed in most colorectal cancer tissues and colon cancer cell lines. Furthermore, the survival and/or the proliferation of the cancer cells including colon cancer cell lines, glioblastoma cell lines, and the like were suppressed *in vitro* in a dose (concentration)-dependent manner with the administration of a small molecular weight type GSK3β inhibitor. The survival and/or the proliferation of the same were similarly suppressed by RNA interference (RNAi) against GSK3β. Moreover, dose-dependent shrinkage of tumor tissues due to administration of the GSK3β inhibitor was also observed in colorectal cancer model animals. Unlike previous knowledge, these results suggest that GSK3β expression and GSK3β activity can be new targets for treatment and prevention of cancer (and particularly colorectal cancer).

Consequently, the present invention relates to a method for suppressing the survival and/or the proliferation of cancer cells by suppressing the activity of this enzyme (GSK3β) through inhibition of the expression of GSK3β or

inhibition of the activation of GSK3β as a result of phosphorylation of GSK3β tyrosine residue at position 216.

The above method can be performed by causing a compound having a structure represented by formula (I) or (II) or a pharmacologically acceptable salt thereof to act on cancer cells.

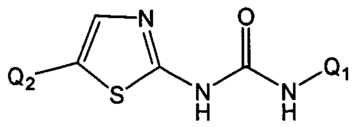

(I)

(wherein $Q_1$ is -($CH_2$) n-Ar-O- (n is an integer between 1 and 5 and Ar is substituted or unsubstituted aryl) and $Q_2$ is a group selected from among halogen, alkyl, hydroxy, alkoxy, aryloxy, haloalkyl, nitro, amino, acylamino, monoalkylamino, dialkylamino, alkylthio, alkylsulfinyl, and alkylsulfonyl)

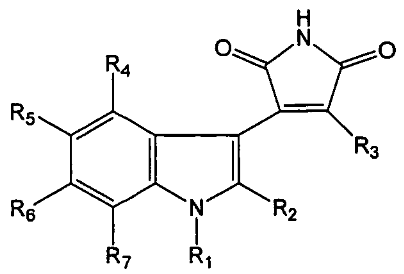

(II)

(wherein $R_1$ is hydrogen, alkyl, aralkyl, alkoxyalkyl, hydroxyalkyl, haloalkyl, aminoalkyl, monoalkylaminoalkyl, dialkylaminoalkyl, azidoalkyl, acylaminoalkyl, alkylsulfonylaminoalkyl, arylsulfonylaminoalkyl, mercaptoalkyl, alkylthioalkyl, glucopyranosil, carboxyalkyl, alkoxycarbonylalkyl, aminocarbonylalkyl, hydroxyalkylthioalkyl, mercaptoalkylthioalkyl, arylthioalkyl, or carboxy alkylthioalkyl,

$R_2$ is hydrogen, alkyl, aralkyl, alkoxyalkyl, hydroxyalkyl, haloalkyl, aminoalkyl, monoalkylaminoalkyl, dialkylaminoalkyl, acylaminoalkyl, alkylsulfonylaminoalkyl, arylsulfonylaminoalkyl, mercaptoalkyl, alkylthioalkyl, carboxyalkyl, alkoxycarbonylalkyl, aminocarbonylalkyl, alkylthio, or alkylsulfinyl,

$R_3$ is a phenyl group substituted with 1, 2, or more substituents selected from the group consisting of halogen, alkyl, hydroxy, alkoxy, haloalkyl, nitro, amino, acylamino, alkylamino, dialkylamino, alkylthio, alkylsulfinyl, and alkylsulfonyl, and

$R_4$, $R_5$, $R_6$, and $R_7$ are each independently hydrogen, halogen, alkyl, hydroxy, alkoxy, aryloxy, haloalkyl, nitro, amino, acylamino, monoalkylamino, dialkylamino, alkylthio, alkylsulfinyl, or alkylsulfonyl).

A preferable example of the above compound is N-(4-methoxybenzyl)-N'-(5-nitro-1,3-thiazol-2-yl) urea or 3-(2,4-dichlorophenyl)-4-(1-methyl-1H-indol-3-yl)-1H-pyrrole-2,5-dione represented by the following formula.

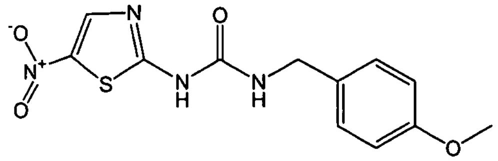

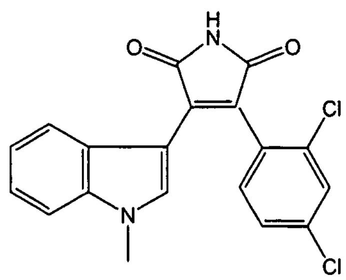

The present invention further provides a pharmaceutical composition for treating cancer comprising the above compound or a pharmacologically acceptable salt thereof as an active ingredient. In this description, the above pharmaceutical composition exerted effects against colorectal cancer, gastric cancer, pancreatic cancer, hepatocellular

carcinoma, and glioblastoma cells and also exerted pharmacologic effects against colon cancer inoculated in nude mice. The effects of the pharmaceutical composition of the present invention are not limited to the above cancers. The pharmaceutical composition is expected to exert its effects against all cancers.

The present invention further provides a method for evaluating the effect of a test substance as an anticancer agent using an effect of inhibiting the activation of this enzyme (GSK3β) as a result of phosphorylation of GSK3β tyrosine residue at position 216 as an indicator.

For example, the above method can be performed by the following steps of:

1) culturing cells under conditions in which a test substance is either added or not added;
2) detecting the expression levels of a GSK3β protein fraction having phosphorylated tyrosine residue at position 216 within the above cells or detecting and determining GSK3β enzyme activity; and
3) evaluating the effect of the test substance as an anticancer agent based on differences in the above expression level of the enzyme or activity under conditions in which the test substance is either added or not added.

In the above method, cells isolated from cancer patients or cells of cancer cell lines including colon cancer cell lines such as SW480, SW620, HT29, LoVo, HCT116, SW48, and RKO and glioblastoma cell lines such as A172, T98G, U251, and U87 can be used.

The method of the present invention can also be performed by the following steps of:

1) maintaining cancer model animal(s) under conditions in which a test substance is either administered or not administered;
2) detecting the expression levels of a GSK3β protein fraction having phosphorylated tyrosine residue at position 216 in the blood or the cells of the above animal(s); and
3) evaluating the effect of the test substance as an anticancer agent based on differences in the above expression level and changes in the growth of inoculated tumors under the conditions in which the test substance is either administered or not administered.

In the above method, animal(s) is preferably a rodent and is particularly preferably a mouse.

Furthermore, in the method of the present invention, protein expression levels can be detected by any one method selected from among a Western blot method, a Dot-blot method, a Slot blot method, an ELISA method, and an RIA method.

According to the present invention, it becomes possible to provide a novel anticancer agent based on the effect of suppressing GSK3β and to perform screening for such anticancer agent. It also becomes possible to perform early diagnosis of cancer using a GSK3β level as an indicator.

Brief Description of the Drawings

Fig. 1 shows: the results (A) of analyzing the expression of total GSK3β, that of phospho-GSK3β$^{Ser9}$ (inactive form), and that of phospho-GSK3β$^{Tyr216}$ (active form) in colon cancer cell lines and human embryonic kidney cells (HEK293 cells) by the Western blot method using specific antibodies (Biosciences and Cell Signaling Technology), respectively; the results (B) of detecting GSK3β activity in colon cancer cell lines and human embryonic kidney cells (HEK293 cells) by *in vitro* kinase assay using a synthetic human β-catenin protein as a substrate (the detection method described in Adler V, et al., Proc Natl Acad Sci U.S.A. 1997, 94: 1686-91 was used); and the results (C) of detecting the activation of protein kinase B (PKB/Akt, which is activated upstream of GSK3β (signal)) in colon cancer cell lines and human embryonic kidney cells (HEK293 cells) using an anti-phospho-Akt $^{Thr308}$ antibody and an anti-phospho-Akt$^{Ser473}$ antibody (Cell Signaling Technology).

Fig. 2 shows the results of observing the expression and localization of total GSK3β (red fluorescence) and phospho-GSK3β$^{Ser9}$ (green fluorescence) in HEK293 cells (upper 4 images) and the SW480 colon cancer cell line (lower 4 images) subjected to double immunofluorescence cytochemical staining. Each scale bar in Fig. 2 corresponds to 100 μm. All images were taken at the same magnification.

Fig. 3 shows the results of detecting the expression levels of total GSK3β, phospho-GSK3β$^{Ser9}$ (inactive form), and phospho-GSK3β$^{Tyr216}$ (active form) fractions in normal mucosa and tumor tissues of colorectal cancer cases. Numerals in the upper column of each panel indicate patient ID numbers (corresponding to Table 2).

N: Non-cancer (normal) mucosa tissue
T: Tumor tissue

Fig. 4 shows the results of analyzing the influence (effect) of AR-A014418 and SB-216763 *in vitro* on the survival, proliferation, and apoptosis of HEK293 cells and various colon cancer cell lines.

A, B, and E: The number of viable cells in each well as measured by MTS assay at points in time at which the indicated lengths of the time (24, 48, 72, and 96 hours) had elapsed since day 0.
C and D: The proportion (D) of proliferating cells' nuclei (PCNA positive) in all the cells observed or the proportion (C) of nuclei undergoing apoptosis in all the cells observed when the cells were subjected to double staining by an immunofluorescence method using an anti-PCNA (proliferating cell nuclear antigen) antibody and Hoechst and then observed under a fluorescence microscope.

Fig. 5 shows the effects of siRNA to target GSK3β on the *in vitro* cell survival rates of HEK293 cells (upper) and SW480 colon cancer cells (lower).
Fig. 6 shows the outline of protocols for experimental therapy trials, which involve administering GSK3β inhibitors to animal models into which colon cancer cells had been inoculated.
Fig. 7 shows the photographs of representative mice (into which colon cancer cells had been inoculated) on week 5 after the start of administration of a GSK3β inhibitor (AR-A014418 or SB-216763) at different doses or DMSO.
Fig. 8 is a graph showing changes over time in inoculated tumor volume after the start of administration of a GSK3β inhibitor (AR-A014418 or SB-216763) at different doses or DMSO to mice into which colon cancer cells had been inoculated.
Fig. 9 shows graphs showing the effects over time of a GSK3β inhibitor (AR-A014418 or SB-216763) with different concentrations or DMSO on the cell survival of various glioblastoma cell lines (T98G, A172, U251, and U87).
Fig. 10 shows the influence of a GSK3β inhibitor (AR-A014418 or SB-216763) on various glioblastoma cell lines (A172, T98G, U251, and U87) and a non-cancer cell line (HEK293). Graph (A) shows the number of tumor cells that had survived 96 hours after treatment with AR-A014418. Graph (B) shows apoptosis induction in tumor cells after treatment with AR-A014418 or SB-216763. Graph (C) shows fractions of cells at the proliferation phase (amount of bromodeoxy-uridine [BrdU] incorporated) after treatment with AR-A014418 or SB-216763.
Fig. 11 shows the results of comparing the influence of a 25 μM GSK3β inhibitor (AR-A014418 or SB-216763) on the cellular senescence of each colon cancer cell line (SW480 or HCT116) and a non-cancer cell line (HEK293) with the influence in the case of DMSO, followed by examination via x-gal staining (staining with x-gal indicates β-galactosidase activity).
Fig. 12 shows the results of examining changes in the proportion of senescent cells at 72 hours after treatment of each colon cancer cell line (SW480 and HCT116) and a non-cancer cell line (HEK293) with a 25 μM GSK3β inhibitor (AR-A014418 or SB-216763) or DMSO by x-gal staining.
Fig. 13 shows the results of examining the influence of a GSK3β inhibitor (AR-A014418 or SB-216763) on the expression of human telomerase reverse transcriptase (hTERT) in each colon cancer cell line (SW480 and HCT116) and a non-cancer cell line (HEK293).
Fig. 14 shows the results of examining the influence of a GSK3β inhibitor (AR-A014418 or SB-216763) on telomerase activity in each colon cancer cell line (SW480 and HCT116) and non-cancer cell line (HEK293).
Fig. 15 shows the results of examining the influence of the inhibited expression of GSK3β by siRNA transfection on (A) telomerase activity and (B) hTERT mRNA expression in each colon cancer cell line (SW480 and HCT116) and a non-cancer cell line (HEK293).
Fig. 15 shows the results of examining by siRNA transfection (introduction of small molecule interference RNA) (A) telomerase activity and (B) the influence of the inhibited expression of GSK3β on hTERT mRNA expression in each colorectal cancer cell line (SW480 and HCT116) and a non-cancer cell line (HEK293).
Fig. 16 shows the principle and the outline of a non-radioisotopic *in vitro* GSK3β enzyme activity assay (Non-RI *in vitro* kinase assay) according to the present invention.
Fig. 17 shows the results of detecting and comparing GSK3β enzyme activity in various cell lines by non-radioisotopic *in vitro* GSK3β enzyme activity assay.
Fig. 18 shows the results of detecting GSK3β enzyme activity in HEK293 cells and various cultured cancer cell lines: SW480, HCT116, and HT29 (colon cancer); MKN-28, NKPS, TMK-1, and NUGC-4 (gastric cancer); MIA-PaCa-2, Capan-1, and BxPC-3 (pancreatic cancer); and HEP-G2 (hepatocellular carcinoma) by nonradioactive *in vitro* GSK3β enzyme activity assay.

P and C: Specimen (P) prepared by immunoprecipitation using an anti-GSK3β antibody and specimen (C) (negative control) prepared by immunoprecipitation using non-specific mouse IgG instead of the antibody, both from protein extracted from the same type of cell, and then similarly analyzed.

Fig. 19 shows the results of verifying the inhibitory effect of a 25 μM GSK3β inhibitor (AR-A014418 or SB-216763) and a negative control, DMSO, on the enzyme activity of the GSK3β immunoprecipitated from colon cancer cell lines (SW480 and HCT116) by means of the non-radioisotopic *in vitro* GSK3β enzyme activity assay.

This description includes part or all of the contents as disclosed in the description of Japanese Patent Application No. 2005-133, which is a priority document of the present application.

Preferred Embodiments of the Invention

Hereafter, the present invention is described in detail.

1. Cancer treatment using glycogen synthase kinase 3β inhibitor

The present invention provides the use of a glycogen synthase kinase 3β (hereinafter, may also be referred to as "GSK3β") inhibitor as a pharmaceutical composition for treating colorectal cancer.

1.1 GSK3β

GSK3β is a multifunctional protein phosphorylating enzyme acting as a suppression factor that inactivates a series of cellular regulatory proteins including β-catenin proto-oncogene product via phosphorylation of such proteins. The present inventors have discovered for the first time that: (1) enhanced GSK3β expression is observed characteristically in cancer tissues of colorectal cancer patients and colon cancer cell lines; (2) the survival and the proliferation of colon cancer cell lines are suppressed by administration of GSK3β inhibitors in a dose-dependent manner, so that apoptosis is induced; and (3) the proliferation of colon cancer cell lines is suppressed by siRNA for GSK3β. Hence, the present inventors have demonstrated that the GSK3β inhibitors are useful as anticancer agents and that the effect of suppressing GSK3β is useful as an indicator for evaluation of an anticancer agent.

Examples of GSK3β according to the present invention include, in addition to human GSK3β, orthologs thereof such as mouse GSK3β. Human GSK3β is the most preferable in terms of the purpose of the present invention. The sequence information of the GSK3β gene and that of the GSK3β protein are known and are readily available from GenBank, which is a public database. For example, the amino acid sequence of the GSK3β protein corresponding to a mouse GSK3β gene is disclosed under Accession Nos. BC060743 and AAH60743.1 or BC006936 and AAH06936.1. Furthermore, the amino acid sequence of the GSK3β protein corresponding to a human GSK3β gene is disclosed under Accession Nos. NM_002093 and NP_002084. These sequences are obviously examples and the sequence of the GSK3β gene and that of the GSK3β protein of the present invention are not limited to these sequences.

1.2 GSK3β inhibitor

In this description, "GSK3β inhibitor" means a substance that physically or chemically inhibits the functions of GSK3β. Many compounds have already been identified as GSK3β inhibitors, and they vary greatly in structure and action mechanism (see Meijer L, Flajolet M, Greengard P. Trends in Pharmacol Sci 2004; 25 (9): 471-480).

The structures of and methods for producing such GSK3β inhibitors are disclosed in detail in the following patent applications or registered patent publications: pyrazolopyrimidine disclosed in JP Patent Publication (Kohyo) No. 2005-536517; thiazolidinedithione derivatives disclosed in JP Patent Publication (Kohyo) No. 2005-535593; dihydropyrazolopyridine compounds disclosed in JP Patent Publication (Kohyo) No. 2005-534713; azole methylidene cyanide derivatives disclosed in JP Patent Publication (Kohyo) No. 2005-533066; a cisindolyl-maleimide derivatives disclosed in JP Patent Publication (Kohyo) No. 2005-531609, 2-substituted-1,3-thiazole compounds disclosed in JP Patent Publication (Kohyo) No. 2005-526835; morpholine derivatives disclosed in JP Patent Publication (Kohyo) No. 2005-526814; 1-[alkyl],1-[(heteroaryl)alkyl] and 1-[(aryl)alkyl]-7-(pyrimidin-4-yl)-imidazo[1,2-a]pyrimidin-5(1H)-one derivatives disclosed in JP Patent Publication (Kohyo) No. 2005-519087; heteroaryl-substituted 2-pyridinyl and 2-pyrimidinyl-6,7,8,9-tetrahydropyrimido[1,2-a]pyrimidin-4-one derivatives disclosed in JP Patent Publication (Kohyo) No. 2005-519086; oxindole derivatives disclosed in JP Patent Publication (Kohyo) No. 2005-516961; oxindole derivatives disclosed in JP Patent Publication (Kohyo) No. 2005-516960; quinazolines disclosed in JP Patent Publication (Kohyo) No. 2005-516948; isoquinolines disclosed in JP Patent Publication (Kohyo) No. 2005-513082; hydroxyalkyl indolocarbazole compounds disclosed in JP Patent Publication (Kohyo) No. 2005-509641; aryl amine disclosed in JP Patent Publication (Kohyo) No. 2005-505515; substituted 2-pyridinyl-6,7,8,9-tetrahydropyrimido[1,2-a]pyrimidin-4-one and 7-pyridinyl-2,3-dihydroimidazo[1,2-a]pyrimidin-5(1H)one derivatives disclosed in JP Patent Publication (Kohyo) No. 2005-504810 and JP Patent Publication (Kohyo) No. 2005-504809; dihydropyrazolopyridine compounds disclosed in JP Patent Publication (Kohyo) No. 2005-501800; 2,4-diamino thiazole derivatives disclosed in JP Patent Publication (Kohyo) No. 2004-53831; 4-(4-methoxybenzyl)-N'-(5-nitro-1,3-thiazol-2-yl) urea disclosed in JP Patent Publication (Kohyo) No. 2004-536111; heterocyclic amine disclosed in JP Patent Publication (Kohyo) No. 2004-536110; pyrimidine compounds disclosed in JP Patent

Publication (Kohyo) No. 2004-527486; 2-allyl amino-pyrimidine disclosed in JP Patent Publication (Kohyo) No. 2004-522777; triazines disclosed in JP Patent Publication (Kohyo) No. 2004-516297; indazolyl-substituted pyrroline compounds disclosed in JP Patent Publication (Kohyo) No. 2004-515499; 3-indolyl-4-phenyl-1H-pyrrole-2,5-dione derivatives disclosed in JP Patent Publication (Kohyo) No. 2004-505078; 2-amino-3-(alkyl)-pyrimidone derivatives disclosed in JP Patent Publication (Kohyo) No. 2003-528095; pyrimidine or pyridines disclosed in JP Patent Publication (Kohyo) No. 2003-527303; pyrazine derivatives disclosed in JP Patent Publication (Kohyo) No. 2003-516974; pyrrole-2,5-diones disclosed in JP Patent Publication (Kohyo) No. 2002-527419; aromatic amido compounds disclosed in JP Patent Republication (Saikohyo) No. 01/081345; and pyrroles disclosed in JP Patent Publication (Kohyo) No. H01-233281 A (1989), for example. Persons skilled in the art will be able to produce and use desired GSK3β inhibitors based on these descriptions.

GSK3β activity is controlled at various stages: (a) posttranscriptional modification (posttranscriptional regulation via phosphorylation of serine residue at position 9 and tyrosine residue at position 216), (b) interaction with protein complexes, (c) substrate priming, and (d) intracellular distribution. It is considered that the above GSK3β inhibitors act on GSK3β at any one of these stages (see Meijer L, Flajolet M, Greengard P. Trends Pharmacol Sci 2004; 25 (9), 471-480).

The present inventors have demonstrated both *in vivo* and *in vitro* that two typical GSK3β inhibitors (AR-A014418 and SB-216763) have a specific suppressive effect against cancer. The present inventors have further confirmed that these GSK3β inhibitors can inhibit the activity of GSK3β that has been subjected to posttranscriptional modification (phosphorylation) in cancer cells.

(1) AR-A014418

AR-A014418 is a thiazole derivative represented by the following formula (see CAS NO: 487021-52-3, WO2003004478, JP Patent Publication (Kohyo) No. 2004-536111).

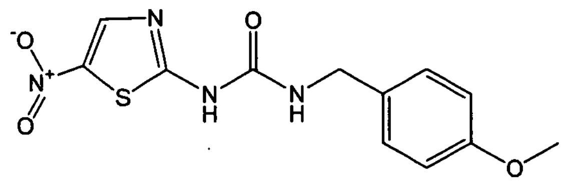

AR-A014418 is marketed as a reagent by Calbiochem or the like and is generally available.

Examples of AR-A014418 are not limited to the above compound. A thiazole derivative represented by the following general formula (I) can also be used as the pharmaceutical composition for suppressing the proliferation of cancer cells and for treating cancer according to the present invention in a manner similar to AR-A014418, as long as it has a similar effect of inhibiting GSK3β.

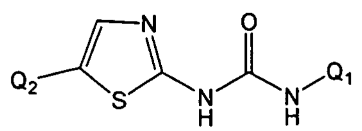

(I)

In formula (I), $Q_1$ is $-(CH_2)$ n-Ar-O- (n is an integer between 1 and 5 and Ar is substituted or unsubstituted aryl), $Q_2$ is a group selected from among halogen, alkyl, hydroxy, alkoxy, aryloxy, haloalkyl, nitro, amino, acylamino, monoalkylamino, dialkylamino, alkylthio, alkylsulfinyl, and alkylsulfonyl.

(2) SB-216763

SB-216763 is referred to as the chemical name of 3-(2, 4-Dichlorophenyl)-4-(1-methyl-1H-indol-3-yl)-1H-pyrrole-2, 5-dione. SB-216763 is a pyrrole derivative represented by the following formula (see EP328026 and Example 45 in JP Patent Publication (Kohyo) No. 01-233281 A (1989)).

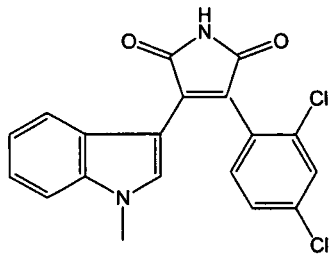

SB-216763 is also marketed as a reagent by Sigma-Aldrich or the like and is generally available.

Examples of SB-216763 are not limited to the above compound. Pyrroles represented by the following general formula (II) can also be used as the pharmaceutical composition for suppressing the proliferation of cancer cells and for treating cancer according to the present invention in a manner similar to SB-216763, as long as it has a similar effect of inhibiting GSK3β.

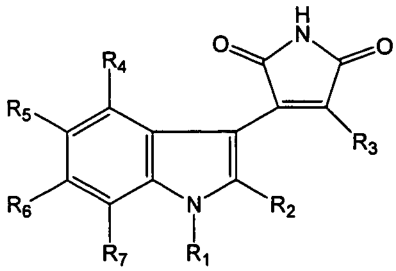

(II)

(In general formula (II), $R_1$ is hydrogen, alkyl, aralkyl, alkoxyalkyl, hydroxyalkyl, haloalkyl, aminoalkyl, monoalkylaminoalkyl, dialkylaminoalkyl, azidoalkyl, acylaminoalkyl, alkylsulfonylaminoalkyl, arylsulfonylaminoalkyl, mercaptoalkyl, alkylthioalkyl, glucopyranosil, carboxyalkyl, alkoxycarbonylalkyl, aminocarbonylalkyl, hydroxyalkylthioalkyl, mercaptoalkylthioalkyl, arylthioalkyl, or carboxyalkylthioalkyl, $R_2$ is hydrogen, alkyl, aralkyl, alkoxyalkyl, hydroxyalkyl, haloalkyl, aminoalkyl, monoalkylaminoalkyl, dialkylaminoalkyl, acylaminoalkyl, alkylsulfonylaminoalkyl, arylsulfonylaminoalkyl, mercaptoalkyl, alkylthioalkyl, carboxyalkyl, alkoxycarbonylalkyl, aminocarbonylalkyl, alkylthio, or alkylsulfinyl,

$R_3$ is a phenyl group substituted with 1, 2, or more substituents selected from the group consisting of halogen, alkyl, hydroxy, alkoxy, haloalkyl, nitro, amino, acylamino, alkylamino, dialkylamino, alkylthio, alkylsulfinyl, and alkylsulfonyl, $R_4$, $R_5$, $R_6$, and $R_7$ are each independently hydrogen, halogen, alkyl, hydroxy, alkoxy, aryloxy, haloalkyl, nitro, amino, acylamino, monoalkylamino, dialkylamino, alkylthio, alkylsulfinyl, or alkylsulfonyl.)

Pharmaceutically acceptable salts of the above compounds such as a basic acid addition salt, an inorganic or organic acid or alkali metal salt, and an alkaline earth metal salt can also be used for suppressing the proliferation of cancer cells and for the pharmaceutical composition according to the present invention.

1.3 GSK3β phosphorylation and cancer treatment

The physiological activity of GSK3β is regulated via phosphorylation (of serine residue at position 9 (inactive form) or tyrosine residue at position 216 (active form)). GSK3β expression and GSK3β activity were enhanced in colorectal cancer cells and tissues regardless of β-catenin activation (nuclear accumulation). The control of the enzyme activity via phosphorylation (inactive form) of serine residue at position 9, which had been verified in non-cancer cells and tissues, was not observed in cancer cells or tissues. Also, a GSK3β (active form) fraction having phosphorylated tyrosine residue at position 216 was expressed in high frequencies in a manner specific to cancer cells and cancer tissues.

When the GSK3β activity of cultured cells was suppressed using a pharmacological inhibitor (AR-A014418 or SB-216763), apoptosis was induced in cancer cells and proliferation of cancer cells was inhibited in a manner depending on the dose of the inhibitor. However, no such effect was observed in the case of a non-cancer cell line (HEK293). When GSK3β expression was knocked down by an RNA interference method, the survival and the proliferation of cancer cells were exclusively suppressed in a manner similar to the effects of the pharmacological inhibitors. A novel oncogenic mechanism was revealed by the above results such that enhancement in GSK3β activity, and particularly the high-level

expression of the GSK3β (active form) fraction having phosphorylated tyrosine residue at position 216, lead to the maintenance and promotion of the survival and/or the proliferation of cancer cells.

Accordingly, it was demonstrated for the first time that cancer treatment and cancer prevention are made possible by modifying GSK3β activity that has been activated by the phosphorylation of tyrosine residue at position 216 through the use of pharmacological and transcriptional control techniques.

2. Method for evaluating the effect of test substance as anticancer agent

The present invention provides a method for evaluating the effect of a test substance as an anticancer agent using as an indicator an inhibitory effect against the GSK3β gene or the GSK3β protein and particularly an inhibitory effect against the activity of this enzyme that has been activated via phosphorylation of tyrosine residue at position 216.

The above method may be used for comparing an inhibitory effect exerted by one test substance under conditions in which it is administered (added) with that exerted by the same under conditions in which it is not administered (not added), followed by evaluation. The above method may also be used for comparing and evaluating similar effects of 2 or more test substances. Alternatively, if correlation between the expression level or the activity of the above gene or protein and an anti-tumor effect is experimentally established, the method may also be used for making an absolute evaluation without comparison with controls based on such correlation.

In the evaluation method of the present invention, an inhibitory effect against the GSK3β gene or the GSK3β protein may also be evaluated using the expression level of the GSK3β gene or the GSK3β protein, or it may also be evaluated using the activity (including stability) of the GSK3β gene or the GSK3β protein as an indicator. Moreover, the evaluation system may be an *in vivo* system or an *in vitro* system. Hereinafter, specific methods for implementing the evaluation method of the present invention will be explained.

2.1 Cultured cell

The cells to be used in the evaluation method of the present invention are not particularly limited, as long they are mammalian cells that express the GSK3β gene at high levels. Cancer cells derived from mammals (preferably, primary cultured cells) and established cancer cell lines are preferable. As the above mammals, humans, mice, rats, hamsters, or the like are preferable. Humans or mice are more preferable. Moreover, artificially transformed cells that may also be used herein are cells that have been recombined via introduction of the GSK3β gene together with its promoter region into the cells so as to express the gene at high levels.

Cells are cultured under conditions in which a test substance is either added or not added. The culture method to be employed herein is not particularly limited and a culture method appropriate for the cells in question may be adequately selected. The method for adding a test substance to cultured cells and the amount of a test substance to be added are also not particularly limited. At an appropriate time point before culturing or during the period for culturing the above cells, a test substance is added to a culture medium and then cultured for a specific period of time. The culture period following the addition of a test substance is not particularly limited and preferably ranges from 30 minutes to 24 hours.

2.2 Cancer model animal

The cancer model animals to be used in the methods of the present invention are not particularly limited, as long as they are non-human mammals. The cancer model animals are preferably rodents and particularly preferably mice. Such cancer model animals may be commercially available cancer model animals or may be produced in accordance with a known method. For example, a colorectal cancer model mouse can be produced in accordance with a previous report (Gupta RA., et al., Activation of nuclear hormone receptor peroxisome proliferator-activated receptor-δ accelerates intestinal adenoma growth, Nat Med 2004, 10(3): 245-247).

The above animals are maintained for an appropriate period under conditions in which a test substance is either administered or not administered. The dose of a test substance to be administered to an animal is not particularly limited. The dose may be adequately determined depending on the nature of the test substance and the body weight of the animal in question. Moreover, the method or the period for administering the test substance to animals is also not particularly limited. The route or the period for administration is adequately determined depending on the nature of the test substance in question.

The blood or the cells of the above animals maintained under conditions in which a test substance is either administered or not administered are isolated. The expression level of the GSK3β gene or the GSK3β protein in the blood or the cells is detected. As cells to be subjected to detection, cells of an affected area, that is, cells from cancer tissue, are preferable.

2.3 Detection of GSK3β gene

An example of a method for detecting the GSK3β gene is a method that involves extracting total RNA from isolated blood or cells and then detecting the expression level of mRNA in the total RNA.

The expression level of the GSK3β gene in total RNA can be detected by preparing cRNA or cDNA from the obtained total RNA, labeling the cRNA or cDNA with an appropriate labeling compound, and then detecting the signal intensity of the labeling compound. The method for detecting the expression level of a gene is not particularly limited. Such method may be adequately selected from known methods described below and then used.

Analysis using immobilization samples such as gene chips, arrays, or membrane filters can be conveniently conducted using a commercial GeneChip (Affimetrix), IntelliGene (TAKARA SHUZO), or filter-made Microarray Atlas System (Clontech) upon which a gene to be detected is immobilized. Alternatively, analysis may also be conducted using a GSK3β immobilization sample prepared based on a known method (Lipshutz RJ, et al., Nat Genet 1999, 21 (suppl) 20-24).

RT-PCR methods or a real-time PCR (TaqMan PCR) method that is one such RT-PCR method is suitable for the evaluation method of the present invention in that such methods are capable of quantitatively detecting a fine amount of RNA with high sensitivity. With the real-time PCR (TaqMan PCR) method, for example, an oligonucleotide probe that hybridizes to a specific region of a target gene is used, wherein the 5' end is labeled with a fluorescent dye (reporter) and the 3' end is labeled with a quenching dye (quencher). When this probe is used under general conditions, fluorescence of the reporter is suppressed by the quencher. Under conditions in which the fluorescence probe can completely hybridize to a target gene, PCR is carried out using TaqDNA polymerase from the outside. As an elongation reaction proceeds via Taq DNA polymerase, the fluorescence probe is hydrolyzed from the 5' end by the exonuclease activity, the reporter dye is liberated, and then fluorescence is emitted. In the real-time PCR method, the initial amount of template DNA is precisely quantified by real-time monitoring of fluorescence intensity.

Examples of methods for analyzing gene expression levels other than the above methods include a subtraction method (Sive HL, John T. St. Nucleic Acids Res 1988; 16: 10937, Wang Z, Brown DD. Proc Natl Acad Sci U.S.A. 1991, 88: 11505-11509), a differential-display method (Liang P, Pardee AB. Science 1992, 257, 967-971, Liang P, Averboukh L, Keyomarsi K, Sager R, Pardee AB. Cancer Res 1992, 52: 6966-6968), a differential·hybridization method (John T. St, Davis RW, Cell 1979, 16: 443-452), and a cross-hybridization method using an appropriate probe ("Molecular Cloning, A Laboratory Manual" Maniatis, T., Fritsch, E.F., Sambrook, J. (1982) Cold Spring Harbor Laboratory Press).

In addition to the above methods, GSK3β gene expression or GSK3β protein expression can also be indirectly detected, under the control of a promoter of the GSK3β gene, using a gene (hereinafter referred to as a "reporter gene") that makes it possible to detect the promoter activity. As reporter genes, chroramphenicol acetyltransferase, firefly luciferase, β-galactosidase, secretory alkaline phosphatase, green-fluorescent protein, and the like can be used, for example.

In addition to test methods using transient gene transfer methods described above, a test method using a host cell subjected to double transformation with a reporter gene and an expression vector of the GSK3β gene can also be employed. In this case, there is a need to establish a cell line using an expression vector such as pIND (produced by Invitrogen Corporation) or pTet-On (produced by Clontech) so that the expression of the reporter gene is promoted under conditions in which the expression of the GSK3β gene is induced. When the obtained cell line is placed under conditions in which the expression of the GSK3β gene is induced, the transcription of the reporter gene is promoted in a manner depending on the expression of the gene. Therefore, the expression level of GSK3β can be evaluated by observing changes in the expression level of a reporter gene between a case where any test substance is added and a case where the same is not added to a medium under conditions in which the expression of the reporter gene is possible.

2.4 Detection of GSK3β protein (GSK3β protein having phosphorylated tyrosine residue at position 216)

The expression level of the GSK3β protein (the GSK3β protein having phosphorylated tyrosine residue at position 216) can be detected using an antibody that specifically binds to the protein. The method for detecting such protein using an antibody is not particularly limited and is preferably any one method selected from among a Western blot method, a Dot-blot method, a Slot blot method, an ELISA method, and an RIA method.

Specimens including blood or cells (used as cell extracts) are prepared as described below as samples for ELISA/RIA or samples for Western blot after removal of insoluble substances via high-speed centrifugation as needed.

As a sample for ELISA/RIA, collected serum is used intact or such serum adequately diluted with a buffer is used, for example. As a sample for Western blot (for electrophoresis), a cell extract is used intact or such cell extract adequately diluted with a buffer and then mixed with a sample buffer (e.g., produced by Sigma-Aldrich) containing 2-mercapto ethanol for SDS-polyacrylamide gel electrophoresis is used, for example. As a sample for Dot/Slot blot, a collected cell extract is used intact or such cell extract adequately diluted with a buffer and then directly adsorbed to a membrane using a blotting apparatus, or the like, is used, for example.

"An antibody (hereinafter, referred to as an "anti-GSK3β antibody") specifically binding to a GSK3β protein (the GSK3β protein having phosphorylated tyrosine residue at position 216)" to be used in this step may also be prepared according to a known method or a commercial antibody may also be used. Such antibody can be obtained in accordance with a conventional technique by immunizing an animal with the GSK3β protein or any polypeptide selected from the amino acid sequence as an antigen, collecting an antibody produced in the animal *in vivo,* and then purifying the antibody. Furthermore, in accordance with a known method (e.g, Kohler and Milstein, Nature 256, 495-497, 1975, Kennet, R. ed., Monoclonal Antibody pp. 365-367, 1980, Prenum Press, N.Y.), a hybridoma is established by fusing an antibody-producing cell producing an anti-GSK3β antibody to a myeloma cell. Thus, a monoclonal antibody can also be obtained from the hybridoma.

Examples of an antigen for antibody preparation include the GSK3β protein or polypeptides each comprising a partial amino acid sequence consisting of at least 6 consecutive amino acids or derivatives thereof prepared by adding any amino acid sequences or carriers (e.g., keyhole limpet hemocyanin that binds to the N-terminus) thereto.

The above antigen polypeptides can be obtained by causing host cells to produce the GSK3β protein through genetic manipulation. Practically, a vector that enables expression of the GSK3β gene is constructed and then the vector is introduced into host cells so as to cause the cells to express the gene.

An anti-GSK3β antibody is directly labeled and then used for detection. Alternatively, the same is designated a primary antibody and then used for detection in conjunction with a labeled secondary antibody that specifically recognizes the primary antibody (recognizes an antibody derived from an animal used for antibody preparation).

Preferable types of the above label include an enzyme (alkaline phosphatase or horseradish peroxidase) or biotin. (However, the use of biotin requires a procedure for further binding an enzyme-labeled streptavidin to the biotin of the secondary antibody.) However, examples of such labels are not limited thereto. As labeled secondary antibodies (or labeled streptavidin), various previously labeled antibodies (or types of streptavidin) are marketed. In addition, in the case of RIA, an antibody labeled with a radioactive isotope such as $^{125}I$ is used and then measurement is performed using a liquid scintillation counter or the like.

Through detection of the activity of the enzyme to be used for labeling, the expression level of an antigen is measured. When labeling with alkaline phosphatase or horseradish peroxidase is performed, substrates that develop color or produce luminescence because of catalysis resulting from these enzymes are marketed.

When a substrate that develops color is used, visual detection is possible using the Western blot method or the Dot/Slot blot method. In the case of the ELISA method, determination is preferably performed by measuring absorbance (wavelengths measured differ depending on substrates) in each well using a commercial microplate reader. Furthermore, a dilution series of an antigen used for the above antibody preparation is prepared. The prepared standard antigen samples and other samples are simultaneously subjected to detection, thereby producing a standard curve through plotting of standard antigen concentrations and measured values. Hence, antigen concentrations in other samples can also be determined.

Meanwhile, when a substrate that produces luminescence is used and the Western blot method or the Dot/Slot blot method is employed, detection is possible by autoradiography using X-ray films or imaging plates or photography using an instant camera. Furthermore, determination using densitometry, a molecular imager Fx system (produced by Bio-Rad Laboratories, Inc), or the like is also possible. Furthermore, when a luminescent substrate is used in the ELISA method, enzyme activity is determined using a luminescence microplate reader (e.g., a reader produced by Bio-Rad Laboratories, Inc).

2.5 Evaluation of test substance

The effect of a test substance as an anticancer agent is evaluated based on differences in the expression level of the GSK3β gene or the GSK3β protein (the GSK3β protein having phosphorylated tyrosine residue at position 216) between a case in which the test substance is administered (added) and a case in which the same is not administered (not added).

Consequently, when the expression level of the GSK3β gene or the GSK3β protein (the GSK3β protein having phosphorylated tyrosine residue at position 216) is significantly reduced under conditions in which the test substance has been administered compared with the same under conditions in which the test substance has not been administered, the test substance can be evaluated as being useful as an anticancer agent. Here, "significantly reduced" means the presence of a statistically significant difference ($p < 0.05$) in terms of the expression level of the GSK3β gene or the GSK3β protein (the GSK3β protein having phosphorylated tyrosine residue at position 216) between conditions in which the test substance has been administered and conditions in which the same has not been administered.

2.6 Non-radioisotopic *in vitro* GSK3β enzyme activity assay (Non-RI *in vitro* kinase assay)

The physiological activity of GSK3β is regulated by phosphorylation (serine residue at position 9 (inactive form)

and tyrosine residue at position 216 (active form)). Therefore, GSK3β activity in each cell can be detected or estimated by either *in vitro* kinase assay or immunoblotting using an antibody specific to a peptide having phosphorylated tyrosine residue at position 216. Moreover, the expression level of a GSK3β protein fraction having phosphorylated tyrosine residue at position 216 merely reflects relatively (or indirectly) the GSK3β enzyme activity. It can be said that techniques for directly detecting and determining GSK3β activity are important in the method for evaluating the effect of inhibiting GSK3β activity in the present invention. The present inventors have developed a non-radioisotopic *in vitro* GSK3β enzyme activity assay as a novel means for determining GSK3β activity.

The outline of the non-radioisotopic *in vitro* GSK3β enzyme activity assay is as shown in Fig. 16. First, a specific anti-GSK3β antibody is added to a given amount of a cell extract, so that the GSK3β protein is immunoprecipitated ("S" in Fig. 16). As a control, non-specific IgG (IgG of an animal species with which an anti-GSK3β antibody has been prepared, such as mouse IgG) is added to the above cell extract in a concentration that is the same as that of the cell extract ("C" in Fig. 16). After centrifugation, the cell extract in the supernatant is removed and then the immunoprecipitate is sufficiently washed with a phosphate buffered saline and a buffer for phosphorylation reaction. Subsequently, in the presence of the buffer for a phosphorylation reaction, a recombinant human β-catenin protein (substrate) and (nonradioactive) ATP are added, followed by a phosphorylation reaction. Next, a sample buffer for SDS-polyacrylamide gel electrophoresis is added to the reaction solution, followed by heating and degeneration. The obtained product is analyzed by Western blotting. The phosphorylated recombinant human β-catenin protein is detected by using an anti-phosphorylated β-catenin peptides antibody specific to a GSK3β recognition sequence. Based on the degree of phosphorylation of the β-catenin protein (signal intensity detected by Western blotting) as detected by this technique, GSK3β activity in the cell (in the cell extract) can be determined. Immobilization of a recombinant human β-catenin protein on beads (resins) or the like via its histidine tags enables on-resins colorimetric assay without Western blotting.

3. Kit for evaluating anticancer agent

An example of the kit for implementing the methods of the present invention is a kit containing at least one of the following a) to f):

a) a set of oligonucleotide primers, each of them with a length of 15 to 30 sequential nucleotides for specific amplification of the GSK3β gene;
b) a polynucleotide probe with a length of 20 to 1500 sequential nucleotides, which specifically binds to the GSK3β gene for detecting the gene;
c) an immobilization sample in which the polynucleotide probe described in b) above has been immobilized;
d) an antibody, which specifically binds to the GSK3β protein (a GSK3β protein having phosphorylated tyrosine residue at position 216) for detecting the GSK3β protein;
e) a secondary antibody capable of specifically binding to the antibody described in d) above; and
f) a GSK3β protein (a GSK3β protein having phosphorylated tyrosine residue at position 216).

The primer described in a) above can be easily designed and then amplified in accordance with a conventional technique such as one using commercial primer design software based on the nucleotide sequence of the above gene.

As the probe described in b) above, when a Northern hybridization method is employed, a single-stranded oligonucleotide or a double stranded DNA with a length of approximately 20 nucleotides is suitably used. When microarrays are used, a double stranded DNA with a length of approximately 100 to 1500 nucleotides or a single-stranded oligonucleotide with a length of approximately 20 to 100 nucleotides is suitably used. When a Gene Chip system (Affymetrix) is used, a single-stranded oligo with a length of approximately 25 nucleotides is preferable. These probes are preferably designed as probes that specifically hybridize to portions with high sequence specificity to the GSK3β gene. These probes or the above primers may be labeled (e.g., via enzyme labeling, radioactive labeling, and fluorescence labeling) with adequate labels. Moreover, they may also be modified with biotin, phosphoric acid, amine, or the like.

The immobilization sample described in c) above is prepared by immobilizing the probe described in b) above on a solid phase such as a glass plate, nylon membrane, micro bead, or silicon chip.

The antibodies described in d) and e) above or the protein in f) can be prepared by the method described in 2.4. The antibodies may be labeled (e.g., via enzyme labeling, radioactive labeling, and fluorescence labeling) with adequate labels or adequately modified with biotin or the like.

In addition to the above components, the kit of the present invention may also include other elements required for implementing the evaluation method of the present invention, such as a reagent for hybridization, for labeling a probe, or for detecting the labeled product, a buffer for reaction, and an enzyme substrate, if necessary.

4. Method for cancer diagnosis

The present inventors have confirmed that the human GSK3β gene and the human GSK3β protein are specifically activated in the diseased tissues of cancer (particularly, colorectal cancer) patients. Therefore, when the expression level of such gene or protein in a specimen isolated from a subject is measured, the onset and the pathological conditions of cancer of the subject can be predicted. The expression level of such gene or protein can be measured basically in accordance with the method described in 2.

Examples:

<Example 1> The influence of GSK3β activity and GSK3β inhibitors in various cell lines and the tumors of colorectal cancer patients

1. Subjects and methods.

Cell lines :

Seven types of colon cancer cell line (Table 1: SW480, SW620, HT29, LoVo, HCT116, SW48, and RKO) and a human embryonic kidney cell line (HEK293) were purchased from the American Type Culture Collection (ATCC, Manassas, VA). The cell lines were cultured and maintained in accordance with recommended protocols. Each cell line was harvested at the exponentially proliferating phase before confluence, washed with phosphate buffered saline (PBS), centrifuged, and then stored at -80°C. Each cell line cultured on a cover glass was fixed with 4% paraformaldehyde, so as to carry out immunocytological staining of GSK3β and phospho-GSK3β$^{Ser9}$.

Colorectal cancer cases and tissue specimens:

Twenty patients (13 males and 7 females with age ranging from 44 to 93 (average of 73 years old)) underwent surgical resection of colorectal cancer at the Kanazawa University Cancer Research Institute Hospital in 2002 and 2003. These patients had agreed to be registered in this study and submitted informed consent forms. One set of a normal mucosa and a tumor tissue specimen collected from a fresh surgical specimen of each patient was immediately frozen using liquid nitrogen and then stored at -80°C. Such a surgical specimen was fixed with 10% neutral buffered formalin and then the fixed specimen was subjected to histopathological examination, as to determie the characteristics of tumor and the stage of tumor in accordance with the TNM classification. A paraffin section representing the histological characteristics of each tumor was analyzed immunohistochemically, so that β-catenin activation was determined.

Western blotting analysis:

Cellular proteins were extracted from the surgical specimens and the cultured cell masses (pellet) that had been cryopreserved, by using a solution (CelLytic™-MT, Sigma-Aldrich) in which protease inhibitors and phosphatase inhibitor (both produced by Sigma-Aldrich) had been mixed therein. The protein concentration of each specimen was measured by the Bradford method using Coomassie Protein Assay Reagent (Pierce).

The extracted protein (100 μg) was separated by SDS-polyacrylamide (10%) gel electrophoresis (SDS-PAGE) and then transferred to nitrocellulose membranes (Amersham). Resultant membranes were caused to react separately with rabbit polyclonal antibodies (Cell Signaling Technology and BD Biosciences) each diluted 1000 folds and then the resulting signals were visualized using a chemiluminescence detection reagent (ECL (trademark) Amersham). Therefore, a GSK3β fraction (phospho-GSK3β$^{Ser9}$) having phosphorylated serine residue at position 9 and a fraction (phospho-GSK3β$^{Tyr216}$) having phosphorylated tyrosine residue at position 216 were separately detected. Subsequently, the resultant membranes were caused to react with a mouse monoclonal antibody (diluted 2500 folds, BD Transduction Laboratories) prepared against N-terminal amino acid residues 1 to 160 of rat GSK3β, as to similarly detect (human) total GSK3β.

Activation of protein kinase B (PKB or Akt, a representative kinase that is activated upstream of GSK3β (signal)) was similarly detected using an antibody (each diluted 1000 folds, Cell Signaling Technology) against phospho-Akt$^{Thr308}$ or phospho-Akt$^{Ser473}$.

*In vitro* kinase assay of GSK3β:

GSK3β was prepared by an immunoprecipitation method from 1 mg of the protein extracted from each cell line. The enzyme activity was detected by *in vitro* kinase assay in accordance with a previous report (Adler V, et al., Proc

Natl Acad Sci U.S.A. 1997, 94: 1686-91) using the recombinant human β-catenin protein as a substrate and $^{32}P$-labeled ATP. The radioactive phosphorylation signal of the β-catenin protein was detected by autoradiography.

Immunofluorescent cytochemical staining:

HEK293 cells and each colon cancer cell line were cultured on cover glasses and then total GSK3β and phospho-GSK3β$^{Ser9}$ were detected by immunofluorescence staining. Antibodies (diluted 100 folds) that had been used in the Western blot method were used as primary antibodies. As secondary antibodies, a Cy3-labeled anti-mouse IgG (H+L) antibody and a FITC-labeled anti-rabbit IgG (H+L) antibody (both produced by Jackson ImmunoResearch), each of which had been diluted 200 folds, were used. Furthermore, Hoechst 33342 (Hoechst) was used for nuclear staining. All techniques for immunofluorescence staining were carried out in accordance with a previous report (Shakoori A, et al., Biochem Biophys Res Commun 2003, 312(3): 850-7). The stained cells were observed and recorded using a fluorescence microscope (Olympus AX80, Olympus) connected to a CCD digital camera (Olympus DP70, Olympus).

Detection of mutations in GSK3β$^{Ser9}$ and K-ras genes:

Genomic DNA was extracted and purified by digestion with proteinase K and treatment with phenol-chloroform from cultured colon cancer cell lines and normal mucosa and tumor tissues collected from surgical specimens. Exon 1 containing codon 9 encoding GSK3β serine residue at position 9 was amplified by PCR using an upstream primer (5'-ATTCGCGAAGAGAGTGATCAT-3': SEQ ID NO: 1) and a downstream primer (5'-CACTGCTAACTTTCATGCTGC-3': SEQ ID NO: 2) through a 35-cycles program of 94°C for 1 minute, 55°C for 1 minute, and 72°C for 1 minute, followed by an elongation reaction of 72°C for 10 minutes.

Mutations in GSK3β codon 9 (TCC) were analyzed by the PCR-RFLP (restriction fragment length polymorphism) method that involves digestion of PCR products with an restriction enzyme *Mnl* I (recognition sequence: CCTC[N]7; New England Biolabs). The restriction enzyme cleaves the PCR products of wild-type codon 9 of 110 base pairs into fragments of 52 and 58 base pairs, respectively, but is unable to cleave PCR products having mutation at the 1st or the 2nd nucleotide of codon 9. Mutations in K-ras gene codons 12 and 13 were detected by PCR-RFLP analysis conducted for PCR products amplified with the mismatch primers using restriction enzymes *Mva* I (Takara) and *Bgl* I (TOYOBO) in accordance with a previous report (Zhang B, et al., Clin Cancer Res 2003, 9(8): 3073-9).

Immunohistochemical analysis of β-catenin activation:

In each case, tissue sections representing the major histological characteristics of colorectal primary tumors were immunohistochemically examined using an avidin-biotin complex method. Activation of oncogenic β-catenin signaling was then determined in accordance with a previous report (Ougolkov AV, et al., Gastroenterology 2002, 122(1): 60-71). An anti-β-catenin antibody (BD Transduction Laboratories) was diluted 100 folds and then used. The results of β-catenin expression in primary tumors were classified based on three types of characteristic pattern recently defined by the present inventors: a cell membrane expression type (M) exerting expression at levels same as those in normal crypt; a difuse nuclear accumulation (NAd) type characterized in that cancer cells showing the nuclear accumulation of β-catenin are observed throughout the tumor; and a nuclear accumulation in the tumor invasion front (NAinv) type characterized in that cancer cells showing the nuclear accumulation of β-catenin are observed to be localized at the tumor invasion front (portion that is in contact with stroma at the deepest part of tumor invasion) and cancer cells in tumor portions other than such invasion front exert β-catenin expression of type M.

Based on the evidence that β-catenin is of a tumorigenic active form (with some exceptions) upon its translocation into the nuclei (Wong NA, et al., Am J Pathol 2002, 160(2): 389-401; and Polakis P, Curr Opin Genet Dev 1999, 9(1): 15-21), it was considered in this study that the nuclear accumulation of β-catenin exerted its tumorigenic activity. Two experienced researchers (AV and BZ) individually determined β-catenin activation patterns in tumors without being informed with information concerning GSK3β activity in subject tumors. Determinations made by the two researchers agreed concerning all tumors.

Cell proliferation analysis:

Each colon cancer cell line and HEK293 cells were seeded at $3 \times 10^3$ cells per well of 96-well culture plates and then cultured overnight in corresponding media (this day was designated day 0). Known GSK3β inhibitors, AR-A014418 (N-(4-methoxybenzyl)-N'-(5-nitro-1, 3-thiazol-2-yl) urea, Calbiochem) and SB-216763 (3-(2, 4-dichlorophenyl)-4-(1-methyl-1H-indol-3-yl)-1H-pyrrole-2, 5-dione, Sigma-Aldrich) were dissolved in dimethyl sulfoxide (DMSO). On day 0, the medium for each cell line were exchanged and then AR-A014418 or SB-216763 was added to the medium to different concentrations (5, 10, 25, and 50 μM). As controls, samples prepared by adding AR-A014418 or SB-216763

to the same concentrations to wells containing no cells and a sample prepared by adding DMSO alone to a well containing cells were analyzed simultaneously.

At time points after indicated lengths of the times (24, 48, 72, and 96 hours) had elapsed from day 0, the number of viable cells in each well was measured using an MTS* viable cell measurement kit (CellTiter 96™ Aqueous, Promega Corp) in accordance with the protocols of Promega. Practically, 20 μL of an MTS mixture solution that had been prepared immediately before addition was added to each well, followed by three hours of reaction at 37°C under 5% $CO_2$ wet conditions. Absorbance at a wavelength of 490 nm was measured using a THERMOmax microplate reader (Molecular Devices). The number of cells of each cell line (in 6 wells for treatment by each concentration of AR-A014418 or SB-216763) was measured, and the average absorbance was calculated to etermine the number of viable cells.
*3-[4, 5-dimethylthiazol-2-yl]-5-[3-carboxy-methoxyphenyl]-2H-tetrazolium inner salt

siRNA transfection (introduction of small interfering RNA) experiment :

A mixture (siRNA SMARTpool™ GSK-3 beta) of 4 types of double stranded siRNA to target human GSK3β and negative control siRNA (siGLO™ RISK-Free siRNA) were synthesized and supplied by Dharmacon RNA Technologies (Lafayette, CO). HEK293 cells and each colon cancer cell line were seeded at $1 \times 10^5$ cells per well on 6-well cell culture plates and then cultured overnight. To assay conditions for realizing the optimum effect of siRNA introduction, GSK3β-specific siRNAs having different concentrations or negative control siRNAs having the same concentration were transfected into cells at approximately 50% confluence in accordance with designated protocols using a TransIT (trademark)-TKO transfection reagent (Mirus Corporation). To examine the effect of siRNA transfection on GSK3β expression, proteins were extracted from cells that had been harvested on day 4 after transfection of respective siRNAs having different concentrations. The proteins were analyzed by the Western blot method using an anti-human GSK3 antibody (diluted 1,000 folds; Upstate Biotechnology) recognizing both GSK3α and GSK3β.

Under the optimum conditions for siRNA transfection, that had been determined by the preliminary experiment, the effect of GSK3β-targeting siRNA transfection on cell proliferation were analyzed by a cell proliferation analysis method in a manner similar to the above section. Practically, HEK293 cells and each colon cancer cell line were seeded at $3 \times 10^3$ cells per well on 96-well cell culture plates and then cultured in corresponding medium overnight. Subsequently, GSK3β siRNA or negative control siRNA with the optimal concentration was transfected into each cell. After indicated times calculated from the day of transfection (24, 48, 72, and 96 hours) had elapsed, the number of viable cells in each well was measured using an MTS cell proliferation analysis kit (Promega).

2. Results

2.1 GSK3β activity in various cell lines

All colon cancer cell lines used in this study showed higher GSK3β expression levels than the level in HEK293 cells. In all the colon cancer cell lines, the expression of an inactive form phspho-GSK3$\beta^{Ser9}$ fraction detectable by the Western blot method using the phspho-GSK3$\beta^{Ser9}$-specific antibody was never observed. On the contrary, the expression level of an active form phospho-GSK3$\beta^{Tyr216}$ fraction was higher in all the colon cancer cell lines (Fig. 1A). Moreover, GSK3β enzyme activity was confirmed in these colon cancer cell lines by *in vitro* kinase assay (Fig. 1B). It was shown by these results that GSK3β was constitutively in activated status in all the colon cancer cell lines.

In contrast to the results observed in the colon cancer cell lines, the expression of a phospho-GSK3$\beta^{Ser9}$ fraction was detected in HEK293 cells. In addition, the present inventors have demonstrated to date that β-catenin is physiologically regulated to undergo stable low-level expression in HEK293 cells (Spiegelman VS, et al., Mol Cell 2000, 5(5): 877-82; and Ougolkov A, et al., J Natl Cancer Inst 2004, 96(15): 1161-70).

As demonstrated by immunofluorescent cytochemical staining, GSK3β was localized in the cytoplasms of HEK293 cells and all the colon cancer cells, while phospho-GSK3$\beta^{Ser9}$ was detected only in HEK293 cells (Fig. 2). The expression levels of total GSK3β and phospho-GSK3$\beta^{Ser9}$ in these cell lines agreed with the results (Fig. 1A) of the Western blot method. The above results showed overexpression of (total) GSK3β, low-level expression of its inactive form, phspho-GSK3$\beta^{Ser9}$ fraction, and high-level expression of its active form, phospho-GSK3$\beta^{Tyr216}$ fraction, in colon cancer cell lines (Table 1), in which β-catenin had been activated via avoidance of GSK3β-mediated phosphorylation by either APC inactivation (SW480, SW620, and LoVo) or β-catenin gene (CTNNB1) mutation (HCT116 and SW48).

(Table 1) Comparison of phosphorylation status of GSK3β and Akt and mutations in APC, CTNNB1, and K-ras genes in various colon cancer cell lines

| | Mutation 12) | | | Phosphorylation | | | |
|---|---|---|---|---|---|---|---|
| Cell lines | *APC* | *CTNNB1*a) | K-*ras*b) | $GSK3\beta^{Ser9}$ | $GSK3\beta^{Tyr216}$ | $Akt^{Tyr308}$ | $Akt^{Ser473}$ |
| SW480 | + | - | 12 | - | + | + | + |
| SW620 | + | - | 12 | - | + | + | - |
| HT29 | + | - | - | - | + | + | - |
| LoVo | + | - | 13 | - | + | + | + |
| HCT116 | - | 45 | 13 | - | + | + | - |
| SW48 | - | 33 | - | - | + | trace | - |
| RKO | N.A. | - | - | - | + | trace | + |

2.2 GSK3β activity in the tumor tissues of colorectal cancer patients

Established culture cell lines adapt to be able to grow for a long time period, so that they may not always reflect the characteristics of the same type of tumor in an actual living body. GSK3β expression, the phosphorylation of serine residue at position 9, and the phosphorylation of tyrosine residue at position 216 in normal colorectal mucosa and tumor tissues collected from 20 colorectal cancer patients (cases) were analyzed. The results obtained by this analysis were subjected to comparison and examination concerning the presence or the absence of β-catenin activation in tumors, the patterns thereof in tumors, and clinical and histopathological factors. As a result, as shown in Fig. 3, the expression of GSK3β and that of the phospho-$GSK3\beta^{Tyr216}$ fraction in tumors in most patients were at higher levels than those in normal mucosa in the same cases. The phospho-$GSK3\beta^{Ser9}$ fraction was detected in high frequencies at high levels in nonneoplastic mucosa. However, in tumors of most cases, excluding case Nos. 74 and 79, such inactive form enzyme fraction was slightly detected or never detected (Table 2).

In the previous reports (shown above), the present inventors have shown the correlation between the overexpression and the nuclear accumulation of β-catenin in colorectal cancer. β-catenin activation was determined by the above method based on such reports and β-catenin nuclear accumulation in tumors. As a result, β-catenin activation was detected in the tumor of 13 cases (65%). Seven of the 13 cases were found to be of the NAd type and 6 of such cases were found to be of the NAinv type. Similar to observations concerning colon cancer cell lines, these results showed that overexpression with a lack of suppressive control of GSK3β is a fundamental characteristic feature of colorectal cancer and is unrelated to the presence or the absence of β-catenin activation or the activation patterns (NAd and NAinv) (Table 2). No specific correlation was observed between GSK3β expression or GSK3β activity in tumors and the clinical characteristics of colorectal cancer cases, including characteristics such as tumor stages and metastasis (Table 2).

(Table 2) Clinical data and molecular characteristics of colorectal cancer cases

| Patient ID | Age/Sex | Site of tumor | TNM/ stage at surgery | Metastasis/ Recurrence[a)] | GSK3β expression | GSK3β phosphorylation | | β-catenin activation | phospho-$Akt^{Thr308}$ | phospho-$Akt^{Ser473}$ | K-*ras*[b)] mutation |
|---|---|---|---|---|---|---|---|---|---|---|---|
| | | | | | | $GSK3\beta^{Ser9}$ | $GSK3\beta^{Tyr216}$ | | | | |
| 31 | 44/F | R | $T_2$ $N_0$ $M_0$ / II | NE | N<T | - | N=T | NAd | - | - | - |
| 59 | 74/M | Tr | $T_2$ $N_1$ $M_0$ / IIIA | NE | N<T | - | N<T | NAinv | + | + | 12 |
| 61 | 83/M | S | $T_2$ $N_0$ $M_0$ / II | NE | N<T | + | N=T | NAd | - | - | - |
| 68 | 62/F | Tr | $T_2$ $N_1$ $M_0$ / IIIA | NE | N<T | trace | N<T | NAd | trace | + | - |
| 70 | 79/M | R | $T_2$ $N_1$ $M_0$ / IIIA | NE | N<T | - | N<T | NAinv | - | - | - |
| 71 | 55/M | A | $T_2$ $N_0$ $M_0$ / II | NE | N<T | - | N<T | NAinv | - | + | - |
| 72 | 93/M | S | $T_2$ $N_0$ $M_0$ / II | NE | N<T | - | N<T | - | + | + | 13 |
| 73 | 80/M | C | $T_2$ $N_0$ $M_0$ / II | NE | N<T | trace | N=T | - | trace | + | - |
| 74 | 70/M | S | $T_2$ No $M_1$ / IV | peritoneum | N<T | + | N<T | NAinv | + | - | - |
| 75 | 73/M | C | $T_2$ $N_0$ $M_0$ / II | NE | N<T | - | N<T | - | trace | + | - |
| 76 | 61/M | R | $T_2$ $N_0$ $M_0$ / II | NE | N<T | - | N=T | - | - | - | - |
| 77 | 78/M | S | $T_2$ $N_0$ $M_0$ / II | NE | N<T | - | N<T | NAd | trace | + | - |
| 78 | 71/F | R | $T_2$ $N_1$ $M_0$ / IIIA | liver | N<T | trace | N<T | NAd | trace | - | 12 |
| 79 | 71/F | A | $T_2$ $N_0$ $M_0$ / II | NE | N>T | + | N<T | - | + | + | 12 |
| 80 | 61/M | Tr | $T_3$ $N_2 M_1$ / IV | liver, peritoneum | N<T | trace | N<T | - | + | trace | - |
| 81 | 71/M | S | $T_3$ $N_1$ $M_0$ / IIIA | NE | N<T | - | N<T | NAinv | - | + | - |

(continued)

| Patient ID | Age/Sex | Site of tumor | TNM/ stage at surgery | Metastasis/ Recurrence[a)] | GSK3β expression | GSK3β phosphorylation | | β-catenin activation | phospho-$Akt^{Thr308}$ | phospho-$Akt^{Ser473}$ | K-*ras*[b)] mutation |
|---|---|---|---|---|---|---|---|---|---|---|---|
| | | | | | | $GSK3\beta^{Ser9}$ | $GSK3\beta^{Tyr216}$ | | | | |
| 82 | 81/F | A | $T_2$ $N_2$ $M_1$ / IV | liver, peritoneum | N<T | - | N<T | NAd | - | - | 12 |
| 83 | 59/M | S | $T_2$ $N_0$ $M_0$ / II | NE | N<T | trace | N<T | - | trace | + | - |
| 84 | 54/F | R | $T_2$ $N_1$ $M_0$ / IIIA | NE | N<T | - | N<T | NAinv | - | - | - |
| 85 | 69/F | R | $T_2$ $N_0$ $M_0$ / II | NE | N=T | - | N<T | NAinv | - | - | - |

Abbreviations in Table 2: F, female; M, male; A, ascending colon; C, cecum; R, rectum; S, sigmoid colon; Tr, transverse colon; N, non-cancer tissue; T, cancer tissue; NE, not evident; -, negative; +, positive; NAd, diffuse-type β-catenin activation; and NAinv, tumor invasion front-type β-catenin activation

2.3 Factors regulating GSK3β activity

Upstream factors (signals) that may regulate GSK3β expression or the inactivation via phosphorylation of its serine residue at position 9 or tyrosine residue at position 216 in colorectal cancer were examined. The best known upstream kinase (phosphorylation enzyme) that inactivates GSK3β is PKB/Akt. This enzyme is activated via phosphorylation in a phosphoinositide 3-kinase (PI3K)-dependent manner. PI3K activation takes place downstream of insulin- and ras-mediated signals. Particularly, the latter signal is activated in a high frequency via activation and mutation of a K-ras gene in colorectal cancer (Adjei AA. J Natl Cancer Inst 2001, 93(14): 1062-74; and Minamoto T, et al., Expert Rev Mol Diag 2002, 2(6): 565-75). In view of such oncogenic signaling networks, active form PKB/Akt (phospho-Akt$^{Thr308}$ and phospho-Akt$^{Ser473}$) and known hot spot mutations (codons 12 and 13) in a K-ras proto-oncogene in colorectal cancer were analyzed. Then, it was examined whether any one of these factors had influence on GSK3β expression or the phosphorylation of its serine residue at position 9 or tyrosine residue at position 216.

Either of active form PKB/Akt fractions was detected in 6 out of 7 colon cancer cell lines and in tumors of 13 out of 20 colorectal cancer cases (Fig. 1C, Tables 1 and 2). Activation of the K-ras gene via mutation thereof was detected in 4 out of 6 colon cancer cell lines (Table 1) and in the tumors of 5 out of 20 colorectal cancer cases (Table 2). In the colon cancer cell lines and the tumors of 20 colorectal cancer cases subjected to this experiment, no apparent correlation was observed between GSK3β activity and the activation of PKB/Akt via phosphorylation or that of K-ras via mutation. These results were obtained because of the lack of phospho-GSK3β$^{Ser9}$ in high frequencies in colon cancer cell lines or the tumors of colorectal cancer cases (Tables 1 and 2). The result showed that upstream factors or molecular mechanisms mediated by unknown signals other than Ras or PKB/Akt suppressed enhancement of expression or inactivation of GSK3β in colorectal cancer.

Recently, it has been reported that through artificial introduction of a mutation by which GSK3β serine residue at position 9 is substituted with another amino acid into codon 9 of the same gene, GSK3β serine residue at position 9 is prevented from being phosphorylated and thus GSK3β is constitutively activated (Wang L, et al., J Biol Chem 2004, 279(31): 32444-52). In this experiment, PCR products amplified from the DNAs of tumor tissue specimens of colon cancer cell lines or colorectal cancer cases were examined by a PCR-RFLP method or sequencing. However, no mutations were detected in GSK3β codon 9 (data not shown). The result showed that a gene mutation that had inhibited the phosphorylation of GSK3β serine residue at position 9 was not a presumptive (or candidate) mechanism for GSK3β activation in colorectal cancer.

2.4 Influence of GSK3β inhibitors on various cell lines

It was examined whether experimental suppression of GSK3β activity and GSK3β expression had an influence on the *in vitro* survival and/or the proliferation of colon cancer cell lines. Colon cancer cell lines (SW480 and HCT116) were treated with AR-A014418 or SB-216763 with different concentrations. The survival (rate) of these cells decreased in a manner depending on the dose (concentration) of each inhibitor (Fig. 4A). The similar effect of the GSK3 β inhibitors was also observed in the other colon cancer cell lines (Fig. 4B and E). However, the effect of these inhibitors to suppress cell survival was not observed in HEK293 cells (Fig. 4A). In colon cancer cells on which the GSK3β inhibitors had been caused to act, apoptosis (observed and measured via Hoechst staining) frequency was increased and cellular proliferation rates (proliferating cell nuclear antigen: measured using PCNA labeling index) were lowered, but these changes were minimal in HEK293 cells (Fig. 4C and D).

Moreover, to elucidate the role of enhanced GSK3β expression in colorectal cancer, the effect of RNA interference (RNAi) to target GSK3β on the survival of individual colon cancer cells was observed. Four types of siRNA (each having final concentration of 25 nM) specific to GSK3β were mixed and then transfected into cells. GSK3β protein expression was specifically decreased in HEK293 cells and colon cancer cells SW480 (Fig. 5). Such selective suppression of GSK3β expression was also supported by the result that GSK3α or actin expression had been unaffected by transfection of either GSK3β-specific siRNA or negative control siRNA (Fig. 5). It is also noteworthy that RNAi to target GSK3β suppressed GSK3β expression in colon cancer cells SW480 and HEK293 cells to the same degree, whereas the RNAi suppressed the survival of colon cancer cells SW480, but did not have influence on the proliferation and the survival of HEK293 cells (Fig. 5). These results supported the specific oncogenic effect of GSK3β overexpressed and activated in colon cancer cells.

3. Conclusion

The results in this example showed enhancement of expression and activation of GSK3β in various colon cancer cell lines and clinical colorectal cancer. The results also showed the significant suppressive effect of the GSK3β inhibitors against colon cancer cell proliferation. These results reversed conventional understanding that GSK3β acts suppressively against known oncogenic signals and thus GSK3β inhibitors are predicted to be potentially carcinogenic.

Consequently, it was shown by this study that novel cancer treatment and prevention are made possible by modifying the activity or the expression of GSK3β, which is constitutively activated in cancer cells via phosphorylation of tyrosine residue at position 216 without phosphorylation of serine residue at position 9, through the use of pharmacological and transcriptional control techniques.

<Example 2> Effect of administration of GSK3β inhibitors to animal models into which colon cancer cells were inoculated

For the purpose of elucidating the antitumor effect of GSK3β inhibition (inhibitors) *in vitro,* an animal experiment was conducted as follows.

1. Subjects and methods

Colon cancer cells SW480 were inoculated subcutaneously in a left axillary region of a six-week-old nude mouse (BALB/cA Jcl, nu/nu, CLEA Japan, Inc) using a 23G (gauge) injection needle. Mice in which the survival of the tumors had been confirmed two weeks after the inoculation of the cancer cells were assigned into 3 groups: AR-A014418-administered group (N = 12), SB-216763-administered group (N = 12), and DMSO-administered (control) group (N = 5). Furthermore, the AR-A014418-administered group and the SB-216763-administered group were each divided into three groups: a 1 mg/kg (corresponding to 5 μM in the cultured cell experiment) group to which 1 mg/kg AR-A014418 or SB-216763 had been administered (N = 4); a 2 mg/kg (corresponding to 10 μM in the cultured cell experiment) group to which 2 mg/kg AR-A014418 or SB-216763 had been administered; and a 5 mg/kg (corresponding to 25 μM in the cultured cell experiment) group to which 5 mg/kg AR-A014418 or SB-216763 had been administered. Then, experimental therapy trials were conducted for a total of six groups of mice (Table 3, Fig. 6). A 0.2 ml aliquot each of AR-A014418 (dissolved in DMSO to have three concentrations: 283 μg/mL = 0.35 mM; 567 μg/mL = 0.7 mM; and 1419 μg/mL = 1.75 mM) and SB-216763 (dissolved in DMSO to have 3 concentrations: 235 μg/mL = 0.35 mM; 471 μg/mL = 0.7 mM; and 1177 μg/mL = 1.75 mM) was injected intraperitoneally three times a week (alternate-day). The same amount of DMSO was also injected intraperitoneally three times a week (alternate-day) to the DMSO-administered group.

(Table 3) Protocols for administration of GSK3β inhibitors to animal models into which colon cancer cells were inoculated

| | | Conditions for administration of GSK3β inhibitors | | |
|---|---|---|---|---|
| | Number of mice | Inhibitor dose | Inhibitor concentration | Intraperitoneal injection |
| Control (DMSO) group | 5 | DMSO alone | | 0.2 mL, 3 times/week (alternate-day) |
| SB-216763-administered group | 4 | 1 mg/kg | 235 μg/mL = 0.35 mM | 0.2 mL, 3 times/week (alternate-day) |
| | 4 | 2 mg/kg | 471 μg/mL = 0.70 mM | 0.2 mL, 3 times/week (alternate-day) |
| | 4 | 5 mg/kg | 1177 μg/mL = 1.75 mM | 0.2 mL, 3 times/week (alternate-day) |
| AR-A014418-administered group | 4 | 1 mg/kg | 283 μg/mL = 0.35 mM | 0.2 mL, 3 times/week (alternate-day) |
| | 4 | 2 mg/kg | 567 μg/mL = 0.70 mM | 0.2 mL, 3 times/week (alternate-day) |
| | 4 | 5 mg/kg | 1419 μg/mL = 1.75 mM | 0.2 mL, 3 times/week (alternate-day) |

The dose of each GSK3β inhibitor was calculated to be equivalent to the concentration of the inhibitor in a culture medium used for cultured cells on the assumption that mouse body fluids account for 70% of the body weight (approximately 20 g). Practically, the inhibitor doses of 1 mg/kg, 2 mg/kg, and 5 mg/kg were equivalent to 5 μM, 10 μM, and 25 μM (concentrations of the inhibitor) in a cell culture medium, respectively, in terms of mouse body fluid volume.

Body weight, feed intake, and size of tumor inoculated (major diameter and minor diameter) were measured every week during the period of administering DMSO or each dose of the inhibitors to mice of each group. On week 5

after administration of DMSO or each inhibitor, the mice were sacrificed via deep ether anesthesia and then tumors inoculated and major organs (lung, liver, pancreas, kidney, and spleen) were removed (Fig. 6). Tumor volume was calculated by the following calculation formula.

$$\text{Tumor volume (cm}^3\text{)} = 0.5 \times \text{major diameter (cm)} \times [\text{minor diameter (cm)}]^2$$

2. Results

Fig. 7 shows representative mice of each group (4 mice per group) on week 5 after the start of the experimental therapy trials. Compared with inoculated tumors of the control (DMSO administered) group, the suppressed growth of subcutaneously inoculated tumors in the left axillary regions was confirmed in all the groups to which the GSK3β inhibitors had been administered. Fig. 8 shows changes over time in tumor volume after administration of different doses of each inhibitor. It was confirmed by this graph that the growth of tumors inoculated had been significantly suppressed in a manner depending on the doses of the GSK3β inhibitors compared with the tumors of the control group. In addition, no clear adverse events were observed in any of the groups, including the control group, in view of feed intake and changes in body weight.

The *in vivo* anti-tumor effect of GSK3β inhibition (inhibitors) was verified by this example for the first time. Furthermore, although the experiment was conducted for a short time period, it was concluded that the GSK3β inhibitors do not have toxicity or cause adverse events.

<Example 3> Effects of GSK3β inhibitors on glioblastoma cells

An important object for the development of cancer therapy based on inhibition of GSK3β (enzyme) is to elucidate if the carcinostatic effect of GSK3β inhibition (inhibitors) is effective against not only colorectal cancer, but also cancer types other than such colorectal cancer and particularly against refractory cancer. Among experiments conducted using cells derived from refractory cancer (e.g., gastric cancer, pancreatic cancer, hepatocellular carcinoma, and brain tumor) other than colorectal cancer, the results of an experiment conducted for glioblastoma as an example of refractory cancer will be described below.

1. Subjects and methods

Glioblastomas are gliomas that are extremely poorly differentiated and are highly malignant and account for approximately 9% of primary brain tumor cases. Complete surgical excision of actual tumors is impossible in most cases. Glioblastomas are resistant to treatment with existing anticancer agents and treatment via irradiation and thus are extremely refractory. As described above, there are no effective therapeutic methods for the tumors. Furthermore, it appears to be the reality that the vital prognosis has not improved over the past 20 to 30 years. Development of a novel therapeutic method therefor is much desired.

In this example, 4 types of glioblastoma cell line (A172, T98G, U251, and U87 purchased from American Type Culture Collection [ATCC, Manassas, VA]) and human embryonic kidney cells HEK293 were used as subjects. The expression of GSK3β and the expression of its fractions that had undergone phosphorylation in these cell lines were analyzed in accordance with <Example 1>. Furthermore, the effects of a known GSK3β inhibitor (AR-A014418 or SB-216763) on the survival, proliferation, and apoptosis of tumor cells were analyzed in a manner similar to that in the case of colon cancer cells.

Each cell line was cultured overnight and then cultured in medium to which AR-A014418 or SB-216763 dissolved in DMSO had been added to predetermined concentrations (5 μM, 10 μM, 25 μM or 40 μM, and 50 μM). Number of viable cells and apoptosis frequencies were measured over time. As controls, number of viable cells and apoptosis frequencies were also measured for cells that had been cultured after addition of the same amount of DMSO. In addition, unlike <Example 1>, a WST-8** assay kit (Cell Counting Kit-8, Wako, Osaka) and a microplate reader produced by Bio-Rad Laboratories, Inc. (Hercules, CA) were used for measurement of number of viable cells. Furthermore, regarding cell fractions during the proliferation phase, the number of cells incorporating bromodeoxy uridine (BrdU) was measured using a Cell Proliferation ELISA BrdU Kit (Roche, Panzberg, Germany), and then the influence of the GSK3β inhibitor (AR-A014418 or SB-216763) on the proliferation of tumor cells was analyzed.

**WST-8: 4-[3-(4-iodophenyl)-2-(4-nitrophenyl)-2H5-tetrazolio]-1, 3-benzene disulfonate

2. Results

As a result of analysis conducted by the Western blot method in a manner similar to that in <Example 1>, GSK3β and its active form fraction having phosphorylated tyrosine residue at position 216 were detected in all the 4 types of glioblastoma cell line (A172, T98G, U251, and U87) (data not shown). GSK3β activity in these glioblastoma cells was verified by the non-radioisotopic *in vitro* enzyme activity assay (see Example 5 described later).

Similar to the case of colon cancer cells, number of viable cells was decreased in a manner depending on the concentration of each inhibitor, and apoptosis was significantly induced in all the glioblastoma cells treated with AR-A014418 or SB-216763, compared with the case (control) in which the cells had been treated with DMSO (Figs. 9, 10A, and 10B). On the other hand, in HEK293 cells, apoptosis was mildly induced by the addition of the GSK3β inhibitor to the medium, but no significant differences were observed compared with the case (control) in which the cells had been treated with DMSO (Fig. 10B). Moreover, proliferation of these glioblastoma cells was suppressed by treatment with the GSK3β inhibitors (Fig. 10C).

It was revealed by the above results that not only in the case of colorectal cancer, but also in the case of glioblastoma, (active form) GSK3β supported the survival and the proliferation of tumor cells so as to prevent apoptosis from occurring. It was thus suggested that (active form) GSK3β is a therapeutic target for treating cancer types in addition to colorectal cancer.

<Example 4> Influence of GSK3β inhibition on immortalization of cancer cells

It is important to elucidate the molecular- and cellular-level mechanisms of the carcinostatic effect of GSK3β inhibition (inhibitors). Hence, the effect of GSK3β inhibition (inhibitors) on cell immortalization, which is one of important biological characteristics common to all types of cancer were analyzed targeting cellular senescence and a mechanism for maintaining telomere.

1. Subjects and methods

Colon cancer cells and non-cancer cells were separately treated with GSK3β inhibitors or specific RNA interference, then cellular-level and molecular-level changes involved in cellular senescence and cell immortalization were analyzed.

In accordance with <Example 1>, each colon cancer cell line (SW480 and HCT116) and a non-cancer cell line (human embryonic kidney cells HEK293) were treated with a 25 μM GSK3β inhibitor (AR-A014418 or SB-216763) or GSK3β-specific RNA interference. As controls, DMSO was administered to these cells or the cells were treated with non-specific RNA interference. Subsequently, the frequency of senescent cells in control cells was compared with that in the corresponding cells in which GSK3β had been inhibited and then the result was analyzed. Furthermore, human telomerase reverse transcriptase (hTERT) mRNA expression that is a molecular indication of cell immortalization and changes in telomerase activity in these cells were compared and analyzed over time.

Senescent cells were determined as cells found to be positive for x-gal staining that is an indicator of β-galactosidase activity. Practically, for this staining, cells cultured on cover glasses were washed with phosphate buffered saline (PBS) and then fixed using a citrate-phosphate fixation solution (0.1 M citric acid, 0.2 M $Na_2HPO_4$, pH 6.0) at room temperature for 5 minutes. Subsequently, cells were washed twice with PBS and then caused to react using a x-gal staining solution (1 mg/mL x-gal [Sigma-Aldrich], 5 mM $K_3Fe[CN]_6$, 2 mM $MgCl_2$, 150 mM NaCl, 30 mM citrate phosphate buffer, and 5 mM $K_4Fe[CN]_6$) at 37°C for 20 hours. Stained cells were washed with PBS and then fixed again with 4% paraformaldehyde. X-gal staining positive cells were identified by observation under a light microscope. The frequency of senescent cells was determided by observing 5 high-power fields and measuring and calculating the average frequency of x-gal staining positive cells in the total number of cells (approximately 100 cells) in each field.

hTERT mRNA expression was analyzed by extracting total RNA from subject cells and then performing the RT-PCR method. An upstream primer (5'-CGGAAGAGTGTCTGGAGCAA-3': SEQ ID NO: 3) and a downstream primer (5'-GGATGAAGCGGAGTCTGGA-3': SEQ ID NO: 4) were used for RT-PCR. The hTERT gene was amplified by performing 36 or 38 cycles of an RT-PCR program (15 seconds at 94°C, 10 seconds at 55°C, and 9 seconds at 72°C). Telomerase activity was detected in accordance with recommended protocols using a commercial kit (TRAPEZE® Telomerase Detection Kit, Chemicon International). hTERT expression levels and telomerase activity in GSK3β-inhibited cells and control cells were determided via measurement and quantification of signals on electrophoresis using a densitometer (NIH Image Software), followed by comparison and analysis thereof.

2. Results

Through 72 hours of treatment with the GSK3β inhibitors (25 μM each), the frequencies of senescent cells that

had been identified as x-gal staining positive cells were significantly increased in all the colon cancer cell lines treated with AR-A014418 or SB-216763 compared with those treated with DMSO. On the other hand, in HEK293 cells, no significant differences were observed between cells treated with any one of the GSK3β inhibitors and cells treated with DMSO in terms of the frequency of senescent cells (Figs. 11 and 12).

Next, the effect of GSK3β inhibition on hTERT expression and telomerase activity was examined. As a result, in the colon cancer cell lines, hTERT expression and telomerase activity were suppressed after 72 or 96 hours of treatment with AR-A014418 or SB-216763. On the other hand, neither changes in hTERT expression nor suppression of telomerase activity due to the GSK3β inhibitors was observed in HEK293 cells (Figs. 13 and 14). Furthermore, through attenuation of GSK3β expression in these cells using specific RNA interference, hTERT expression and telomerase activity were suppressed in the colon cancer cells in a manner similar to the case of the GSK3β inhibitors, but no changes were observed in HEK293 cells (Fig. 15).

It was suggested by the above results that prevention of cancer cell immortalization and promotion of cancer cell senescence can be one of molecular-level and cellular-level mechanisms of the carcinostatic effect of GSK3β inhibition.

<Example 5> Development of non-radioisotopic *in vitro* GSK3β enzyme activity assay (Non-RI *in vitro* kinase assay)

The expression level of a GSK3β protein fraction having phosphorylated tyrosine residue at position 216 merely reflects GSK3β (enzyme) activity relatively (or indirectly). Hence, techniques for directly detecting and determining the enzyme activity are important for evaluation of the effect of existing substances to inhibit GSK3 β activity. Such techniques are useful in the development of methods for diagnosing diseases (e.g., Type II diabetes, Alzheimer's disease, and cancer), the development or the onset of which the enzyme (activity) is involved in. Furthermore, such techniques are also extremely useful in screening for and identifying substances that can have an effect of inhibiting enzyme activity in view of drug development for these diseases. The fact that ATP labeled with a radioactive isotope (e.g., $^{32}P$) is required in conventional methods for determining the activity of not only GSK3β but also other protein phosphoenzymes limits their implementation. Hence, the present inventors developed a non-radioisotopic *in vitro* GSK3β enzyme activity assay as a novel convenient technique for determining GSK3 β activity that requires no radioactive isotopes.

1. Principle

The principle of the non-radioisotopic *in vitro* GSK3β enzyme activity assay is outlined with reference to Fig. 16. First, a specific anti-GSK3β antibody and beads (resins) are added to a cell extract and then the GSK3β protein is immunoprecipitated (S in Fig. 16). As a negative control (control), non-specific mouse IgG with the same concentration is added to the same type of cell extract (C in Fig. 16). Next, in the presence of a kinase buffer, a recombinant human β-catenin protein (substrate) and nonradioactive ATP (P in Fig. 16) are added, and then a phosphorylation reaction is performed. Subsequently, the reaction solution is analyzed by the Western blot method. A phosphorylated human β-catenin protein is detected using an anti-phosphorylate β-catenin peptides antibody. When the recombinant human β-catenin protein is immobilized on beads (resins) or the like via its histidine tags, on-resins colorimetric assay can also be carried out without using the Western blot method.

2. Subjects and methods

In the development of this assay, HEK293 cells and various cultured cancer cell lines (SW480, HCT116, and HT29 (colon cancer); MKN-28, NKPS, TMK-1, and NUGC-4 (gastric cancer); MIA-PaCa-2, Capan-1, and BxPC-3 (pancreatic cancer); and HEP-G2 (hepatocellular carcinoma)) were used. These cells were all purchased from the American Type Culture Collection (ATCC, Manassas, VA) and then cultured in each of their recommended medium and under recommended culture conditions.

In accordance with the methods in <Example 1>, the protein was extracted from each cell line using a cell lysis buffer (0.2 M Tris-HCl pH 7.5, 1.5 M NaCl, 10 mM EDTA, 10 mM EGTA, 10% Triton X-100, 25 mM sodium pyrophosphate, 10 mM β-glyceophosphate, 10 mM $Na_3VO_4$, and Protease inhibitors cocktail [Sigma-Aldrich]). GSK3β was immunoprecipitated from 1 mg (1000 μg) of the cellular protein in accordance with a conventional method using a mouse monoclonal anti-GSK3β antibody (BD Transduction Laboratories) and 20 μL of Protein A/G Agarose resins (Calbiochem). The protein was then purified on the resins (hereinafter, referred to as GSK3β). For a negative control for immunoprecipitation, non-specific mouse IgG was used instead of the anti-GSK3β antibody. Cell-derived GSK3β was washed twice with phosphate buffered saline (PBS) and then washed twice with a phosphorylation reaction buffer (kinase buffer) (0.25 M Tris-HCl pH 7.5, 10 mM β-glyceophosphate, 20 mM dithiothreitol [DTT], 1 mM $Na_3VO_4$, and 0.1 M $MgCl_2$). The cell-derived GSK3β was suspended in the kinase buffer. A 2 μL aliquot of 10 mM ATP (nonradioactive) and 4 μg of the recombinant human β-catenin protein (substrate) were added to the suspension to a total of 50 μL. The kinase reaction solution was caused

to react in a water bath (at 30°C) for 30 minutes. An equivalent amount of 2 × SDS electrophoresis sample buffer was added, the resultant was heated at 95°C for 5 minutes, and thus the phosphorylation reaction was stopped.

The supernatant of the reaction solution was separated by SDS polyacrylamide gel electrophoresis, and the following target proteins were analyzed by the Western blot method in a manner similar to that in <Example 1>. Phosphorylation of the recombinant human β-catenin protein that had been used as a substrate for GSK3β-mediated phosphorylation was detected using a phosphorylated β-catenin peptides (p-β-catenin$^{Ser33/37Thr41}$)-specific antibody (Cell Signaling). Next, GSK3β and the recombinant human β-catenin protein in the reaction solutions were similarly detected by the Western blot method. This also makes it possible to compare the cells in terms of GSK3β expression and relative activity.

The technique was further applied to determine the effect of GSK3β inhibitors to inhibit the enzyme activity. Practically, SB-216763 or AR-A014418 was added to a kinase reaction solution to a final concentration of 25 μM. These reaction solutions were similarly analyzed by the Western blot method. The results were compared with the results in the case of β-catenin phosphorylation where DMSO alone had been added instead of the inhibitors.

2. Results

First, various colon cancer cell lines (SW480, HT29, and HCT116) and a non-cancer cell line (HEK293) were analyzed using the non-radioisotopic *in vitro* GSK3β enzyme activity assay, so that GSK3β enzyme activity was detected in all the cells (Fig. 17). Based on the signal intensity of phosphorylated β-catenin, GSK3β activity of the colon cancer cells was found to be higher than that in HEK293 cells. It was concluded that the result was in agreement with the result of the phospho-GSK3β$^{Tyr216}$ fraction as detected by the Western blot method (Fig. 1A).

Next, various gastrointestinal cancer (including colon cancer) cells were subjected to similar analysis. As a result, in addition to the colon cancer cell lines, GSK3β enzyme activity was specifically detected in gastric cancer cell lines, pancreatic cancer cell lines, and a hepatocellular carcinoma cell line. In particular, strong GSK3β activity was detected in pancreatic cancer cells (BxPC-3) and hepatocellular carcinoma cells (HEP-G2) (Fig. 18). Total expression level of GSK3β together with GSK3β activity in each cell type could be quantitatively compared by this analysis. In cells of the same cell line, the expression level of this enzyme was found to correlate with the activity (Fig. 18).

Furthermore, with the use of this assay, verification of the effect of known GSK3β inhibitors was attempted. As a result, it was confirmed that GSK3β activity in the 2 types of colon cancer cell line (SW480 and HCT116) was completely inhibited by 25 μM SB-216763 or 25 μM AR-A-014418 (Fig. 19).

The usefulness of the nonradioisotopic *in vitro* GSK3β enzyme activity assay was verified by the series of the results. Moreover, it was suggested that if the assay is improved to be an on resins colorimetric assay, the improved method can be applied to the development of methods for disease diagnosis using this enzyme as an indicator, screening for novel substances inhibiting GSK3β activity, and development of techniques for inhibiting this enzyme.

<Example 6> The effect of GSK3β inhibition in gastric cancer cell lines, pancreatic cancer cell lines, and a hepatocellular carcinoma cell line

For the purpose similar to that in <Example 3>, cultured cell lines established from carcinoma of gastrointestinal organs other than colorectal cancer, and particularly from refractory gastrointestinal cancer were subjected to the analysis of GSK3β expression and GSK3β activity. As shown in <Example 5> and Fig. 18A and B, among gastric cancer cell lines MKN-28, NKPS, TMK-1, and NUGC-4, pancreatic cancer cell lines MIAPaCa-2, Capan1, and BxPC-3, and hepatocellular carcinoma HEP-G2 that had been subjected to the analysis, GSK3β expression and GSK3β activity were clearly observed in all the cell lines other than MKN-28 and MIAPaCa-2.

Based on the results of this analysis, the effects of GSK3β inhibition (a small-molecule inhibitor and the RNA interference method) on the survival, proliferation, and apoptosis of these cancer cells were examined by the methods of <Example 1> and <Example 3>. Based on the results of the above analysis, cancer-inoculated animal models were produced using cancer cell lines (e.g., NUGC-4, BxPC-3, and HEP-G2) derived from different organs and exerting high GSK3β activity. The *in vivo* effect (tumor growth suppression) of GSK3β inhibitors against these refractory gastrointestinal cancer cells was examined in accordance with <Example 2>.

All publications, patents, and patent applications cited herein are incorporated herein by reference in their entirety.

Industrial Applicability

The present invention is expected to contribute to the development of novel strategies for cancer treatment and cancer prevention and to development of molecular epidemiological studies for cancer.

Sequence Listing Free Text

SEQ ID NO: 1-explanation of artificial sequence: primer
SEQ ID NO: 2-explanation of artificial sequence: primer
SEQ ID NO: 3-explanation of artificial sequence: primer
SEQ ID NO: 4-explanation of artificial sequence: primer

## Claims

1. A method for suppressing the survival and/or the proliferation of cancer cells through inhibition of the expression of glycogen synthase kinase 3β (GSK3β) or inhibition of the activity of this enzyme (GSK3β) activated as a result of phosphorylation of GSK3β tyrosine residue at position 216.

2. The method according to claim 1, comprising causing a compound having a structure represented by formula (I) or (II) or a pharmacologically acceptable salt thereof to act on the cancer cells:

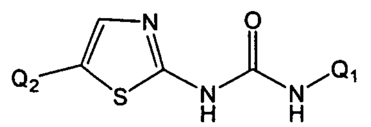

(I)

(wherein $Q_1$ is $-(CH_2)$ n-Ar-O- (n is an integer between 1 and 5 and Ar is substituted or unsubstituted aryl) and $Q_2$ is a group selected from among halogen, alkyl, hydroxy, alkoxy, aryloxy, haloalkyl, nitro, amino, acylamino, monoalkylamino, dialkylamino, alkylthio, alkylsulfinyl, and alkylsulfonyl); or

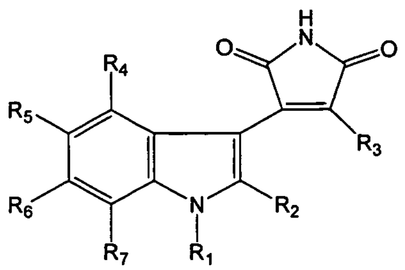

(II)

(wherein $R_1$ is hydrogen, alkyl, aralkyl, alkoxyalkyl, hydroxyalkyl, haloalkyl, aminoalkyl, monoalkylaminoalkyl, dialkylaminoalkyl, azidoalkyl, acylaminoalkyl, alkylsulfonylaminoalkyl, arylsulfonylaminoalkyl, mercaptoalkyl, alkylthioalkyl, glucopyranosil, carboxyalkyl, alkoxycarbonylalkyl, aminocarbonylalkyl, hydroxyalkylthioalkyl, mercaptoalkylthioalkyl, arylthioalkyl, or carboxy alkylthioalkyl,

$R_2$ is hydrogen, alkyl, aralkyl, alkoxyalkyl, hydroxyalkyl, haloalkyl, aminoalkyl, monoalkylaminoalkyl, dialkylaminoalkyl, acylaminoalkyl, alkylsulfonylaminoalkyl, arylsulfonylaminoalkyl, mercaptoalkyl, alkylthioalkyl, carboxyalkyl, alkoxycarbonylalkyl, aminocarbonylalkyl, alkylthio, or alkylsulfinyl,
$R_3$ is a phenyl group substituted with 1, 2, or more substituents selected from the group consisting of halogen, alkyl, hydroxy, alkoxy, haloalkyl, nitro, amino, acylamino, alkylamino, dialkylamino, alkylthio, alkylsulfinyl, and alkylsulfonyl, and
$R_4$, $R_5$, $R_6$, and $R_7$ are each independently hydrogen, halogen, alkyl, hydroxy, alkoxy, aryloxy, haloalkyl, nitro, amino, acylamino, monoalkylamino, dialkylamino, alkylthio, alkylsulfinyl, or alkylsulfonyl).

3. The method according to claim 2, wherein the compound is N-(4-methoxybenzyl)-N'-(5-nitro-1,3-thiazol-2-yl) urea or 3-(2,4-dichlorophenyl)-4-(1-methyl-1H-indol-3-yl)-1H-pyrrole-2,5-dione.

4. The method according to any one of claims 1 to 3, wherein the cancer is at least one selected from among colon cancer, gastric cancer, pancreatic cancer, liver cancer, and glioblastoma.

5. The method according to claim 4, wherein the cancer is colon cancer.

6. A pharmaceutical composition for treating cancer, comprising a compound having a structure represented by formula (I) or (II) or a pharmacologically acceptable salt thereof as an active ingredient:

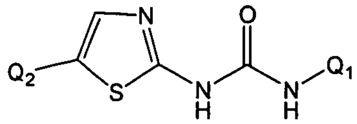

(I)

(wherein $Q_1$ is $-(CH_2)$ n-Ar-O- (n is an integer between 1 and 5 and Ar is substituted or unsubstituted aryl) and $Q_2$ is a group selected from among halogen, alkyl, hydroxy, alkoxy, aryloxy, haloalkyl, nitro, amino, acylamino, monoalkylamino, dialkylamino, alkylthio, alkylsulfinyl, and alkylsulfonyl); or

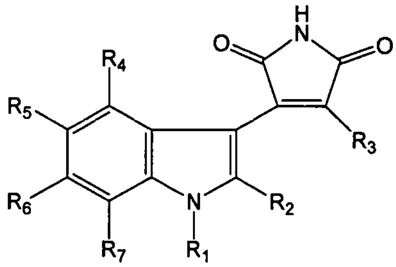

(II)

(wherein $R_1$ is hydrogen, alkyl, aralkyl, alkoxyalkyl, hydroxyalkyl, haloalkyl, aminoalkyl, monoalkylaminoalkyl, dialkylaminoalkyl, azidoalkyl, acylaminoalkyl, alkylsulfonylaminoalkyl, arylsulfonylaminoalkyl, mercaptoalkyl, alkylthioalkyl, glucopyranosil, carboxyalkyl, alkoxycarbonylalkyl, aminocarbonylalkyl, hydroxyalkylthioalkyl, mercaptoalkylthioalkyl, arylthioalkyl, or carboxy alkylthioalkyl,

$R_2$ is hydrogen, alkyl, aralkyl, alkoxyalkyl, hydroxyalkyl, haloalkyl, aminoalkyl, monoalkylaminoalkyl, dialkylaminoalkyl, acylaminoalkyl, alkylsulfonylaminoalkyl, arylsulfonylaminoalkyl, mercaptoalkyl, alkylthioalkyl, carboxyalkyl, alkoxycarbonylalkyl, aminocarbonylalkyl, alkylthio, or alkylsulfinyl,
$R_3$ is a phenyl group substituted with 1, 2, or more substituents selected from the group consisting of halogen, alkyl, hydroxy, alkoxy, haloalkyl, nitro, amino, acylamino, alkylamino, dialkylamino, alkylthio, alkylsulfinyl, and alkylsulfonyl, and
$R_4$, $R_5$, $R_6$, and $R_7$ are each independently hydrogen, halogen, alkyl, hydroxy, alkoxy, aryloxy, haloalkyl, nitro, amino, acylamino, monoalkylamino, dialkylamino, alkylthio, alkylsulfinyl, or alkylsulfonyl).

**7.** The pharmaceutical composition according to claim 6, wherein the compound is N-(4-methoxybenzyl)-N'-(5-nitro-1,3-thiazol-2-yl) urea or 3-(2,4-dichlorophenyl)-4-(1-methyl-1 H-indol-3-yl)-1H-pyrrole-2,5-dione.

**8.** The pharmaceutical composition according to claim 6 or 7, which suppresses the survival and/or the proliferation of cancer cells through inhibition of the activity of glycogen synthase kinase 3β (GSK3β) activated as a result of phosphorylation of GSK3β tyrosine residue at position 216.

**9.** The pharmaceutical composition according to any one of claims 6 to 8, wherein the cancer is at least one selected from among colon cancer, gastric cancer, pancreatic cancer, liver cancer, and glioblastoma.

**10.** The pharmaceutical composition according to claim 9, wherein the cancer is colon cancer.

**11.** A method for evaluating the effect of a test substance as an anticancer agent using as an indicator an effect of inhibiting the activation of glycogen synthase kinase 3β (GSK3β) as a result of phosphorylation of GSK3β tyrosine residue at position 216.

**12.** The method according to claim 11, comprising the steps of:

1) culturing cells under conditions in which a test substance is either added or not added;
2) detecting the expression levels of a GSK3β protein fraction having phosphorylated tyrosine residue at position 216 within the cells or detecting and determining GSK3β enzyme activity; and
3) evaluating the effect of the test substance as an anticancer agent based on differences in the expression

levels or the activity under conditions in which the test substance is either added or not added.

**13.** The method according to claim 12, wherein the cells are isolated from a cancer patient or are cells of a cancer cell line.

**14.** The method according to claim 11, comprising the steps of:

1) maintaining a cancer model animal(s) under conditions in which a test substance is either administered or not administered;
2) detecting the expression levels of a GSK3β protein fraction having phosphorylated tyrosine residue at position 216 in the blood or the cells of the animal(s); and
3) evaluating the effect of the test substance as an anticancer agent based on differences in the expression levels and changes in the growth of inoculated tumors under the conditions in which the test substance is either administered or not administered.

**15.** The method according to claim 14, wherein the animal(s) is a mouse.

**16.** The method according to any one of claims 12 to 15, wherein the expression level of a protein is detected by any one method selected from among a Western blot method, a Dot-blot method, a Slot blot method, an ELISA method, and an RIA method.

Fig. 1

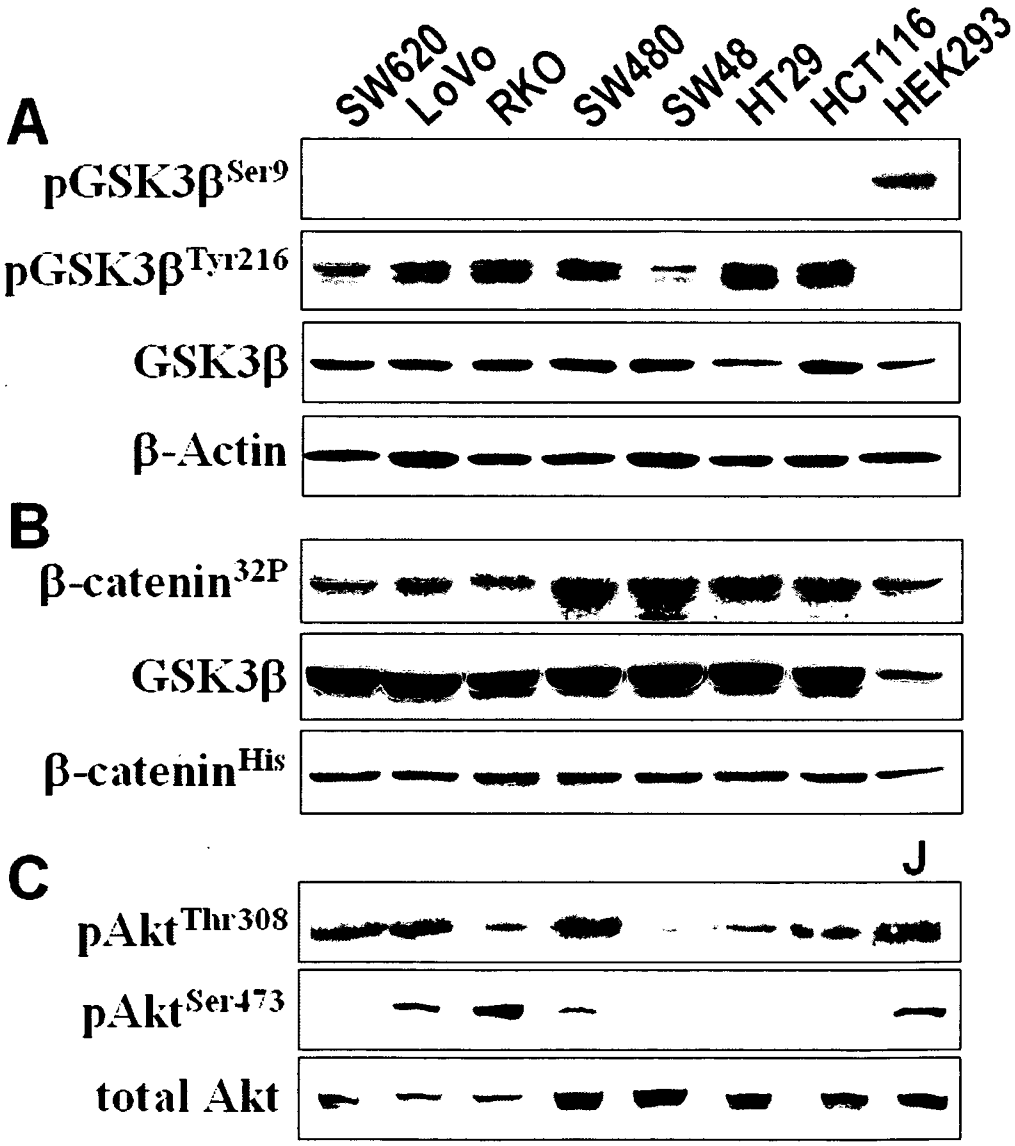
SW620
LoVo
RKO
SW480
SW48
HT29
HCT116
HEK293
A
pGSK3β^Ser9
pGSK3β^Tyr216
GSK3β
β-Actin
B
β-catenin^32P
GSK3β
β-catenin^His
C
J
pAkt^Thr308
pAkt^Ser473
total Akt

Fig. 2

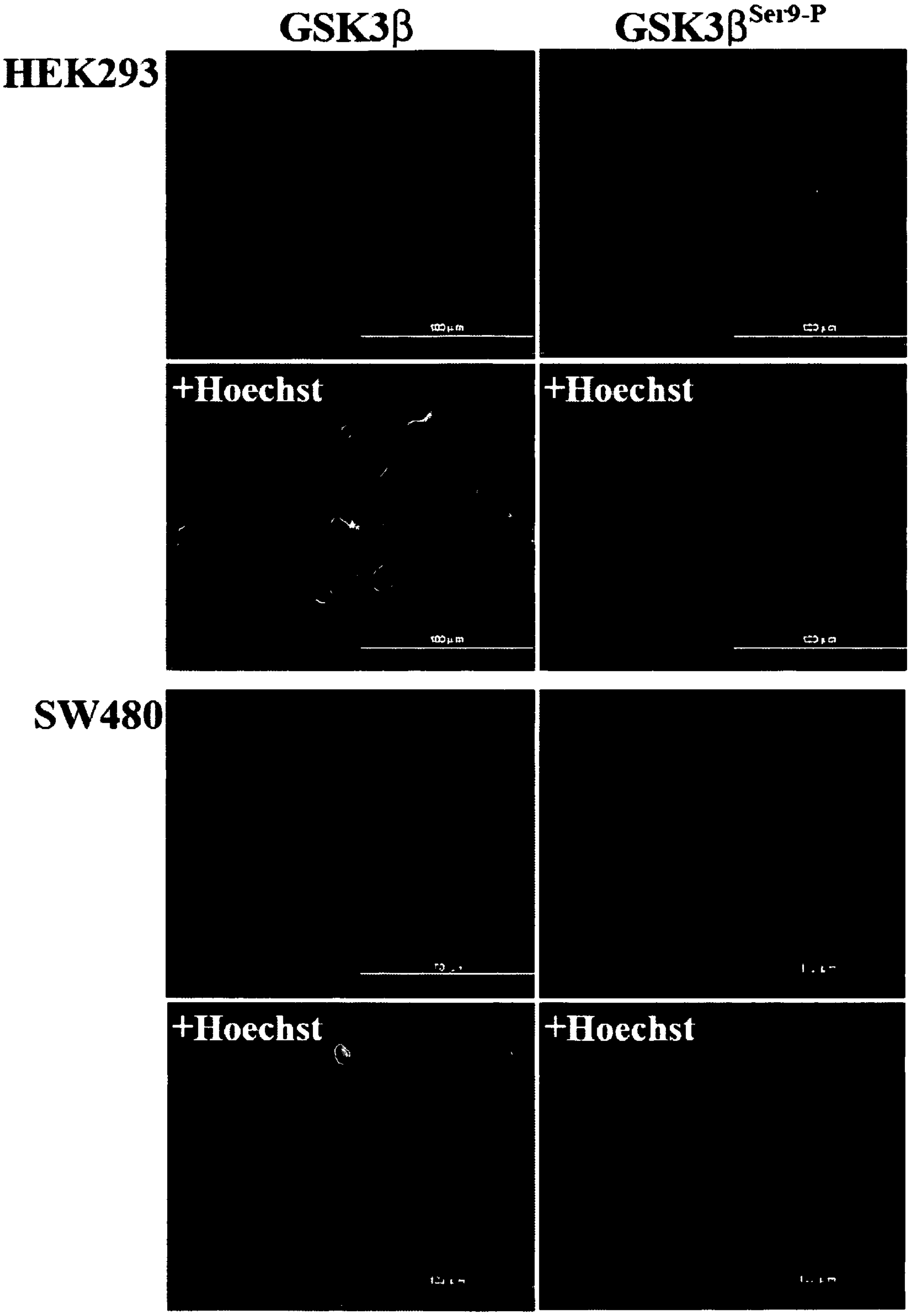
GSK3β
GSK3β$^{Ser9-P}$
HEK293
+Hoechst
+Hoechst
SW480
+Hoechst
+Hoechst

Fig. 3

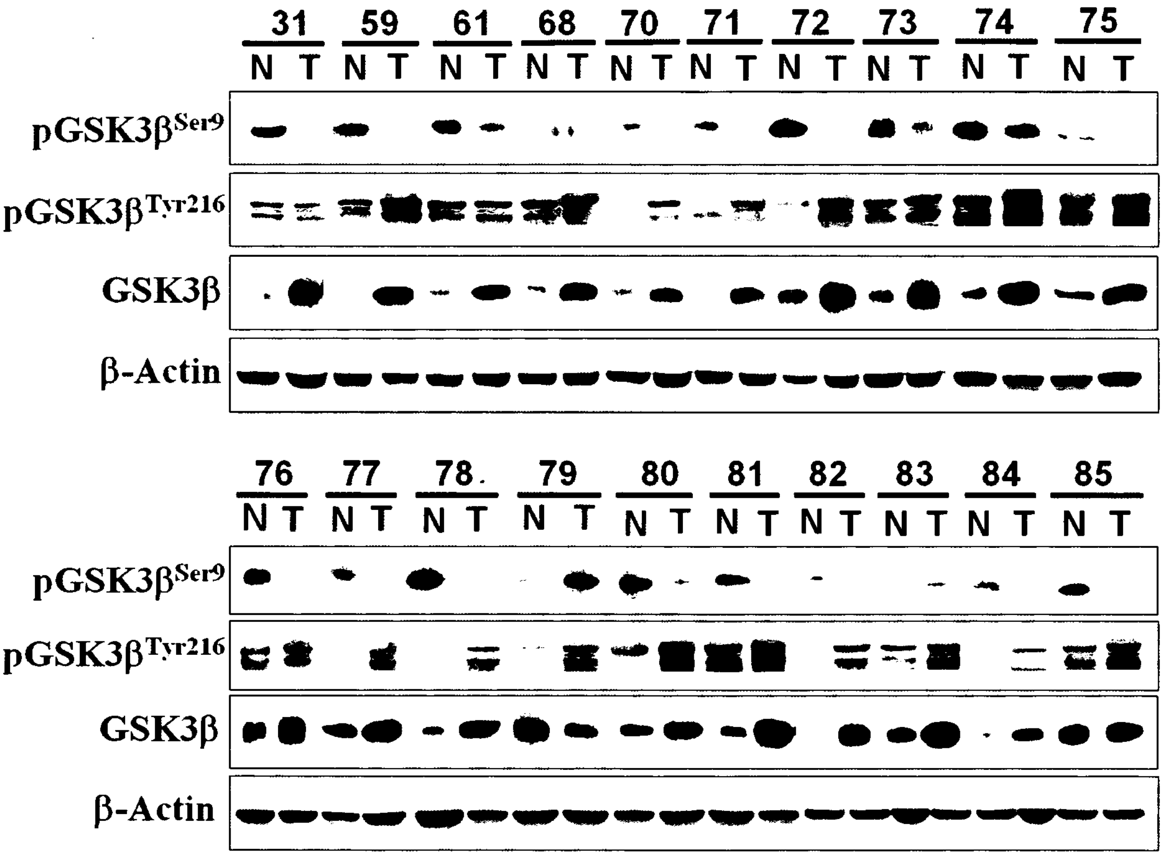
31 59 61 68 70 71 72 73 74 75
N T N T N T N T N T N T N T N T N T N T
pGSK3β Ser9
pGSK3β Tyr216
GSK3β
β-Actin
76 77 78 79 80 81 82 83 84 85
N T N T N T N T N T N T N T N T N T N T
pGSK3β Ser9
pGSK3β Tyr216
GSK3β
β-Actin

Fig. 4

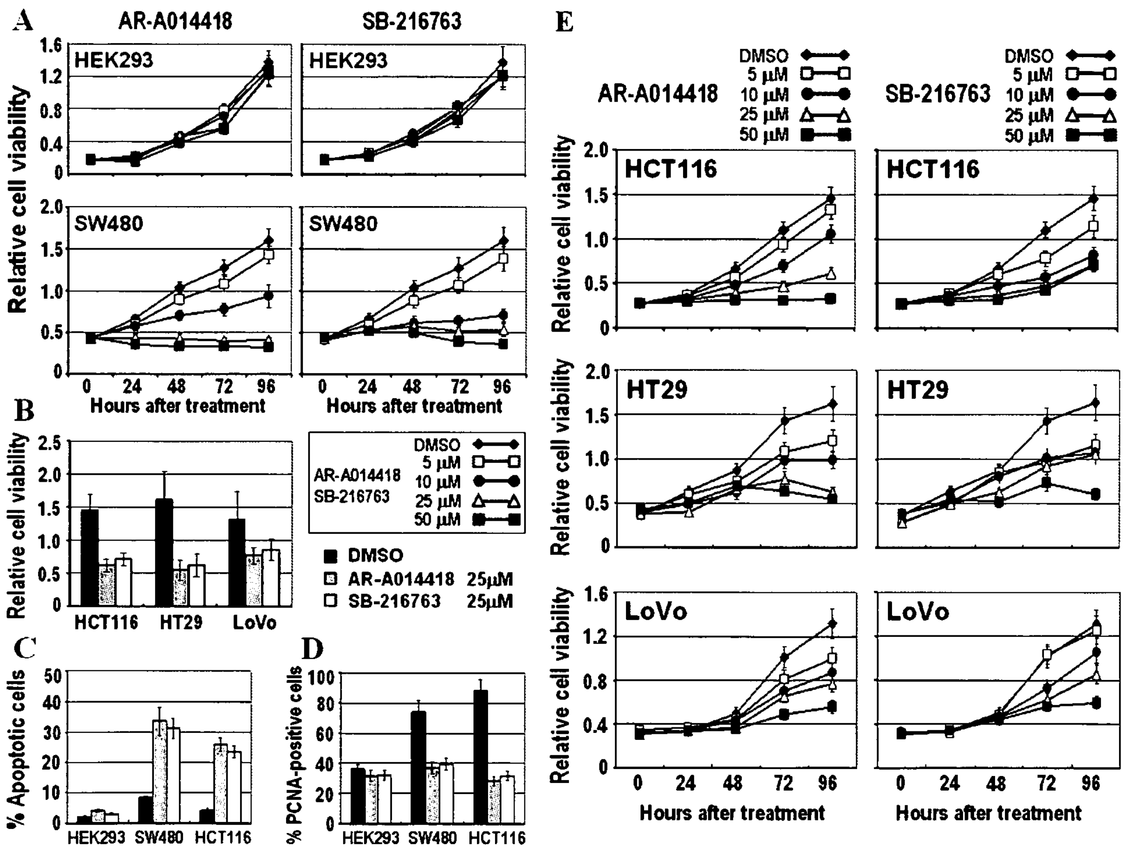
A
AR-A014418
SB-216763
HEK293
SW480
Relative cell viability
Hours after treatment
B
HCT116
HT29
LoVo
DMSO
AR-A014418 25μM
SB-216763 25μM
5 μM
10 μM
25 μM
50 μM
C
% Apoptotic cells
HEK293
SW480
HCT116
D
% PCNA-positive cells
E
HCT116
HT29
LoVo

Fig. 5

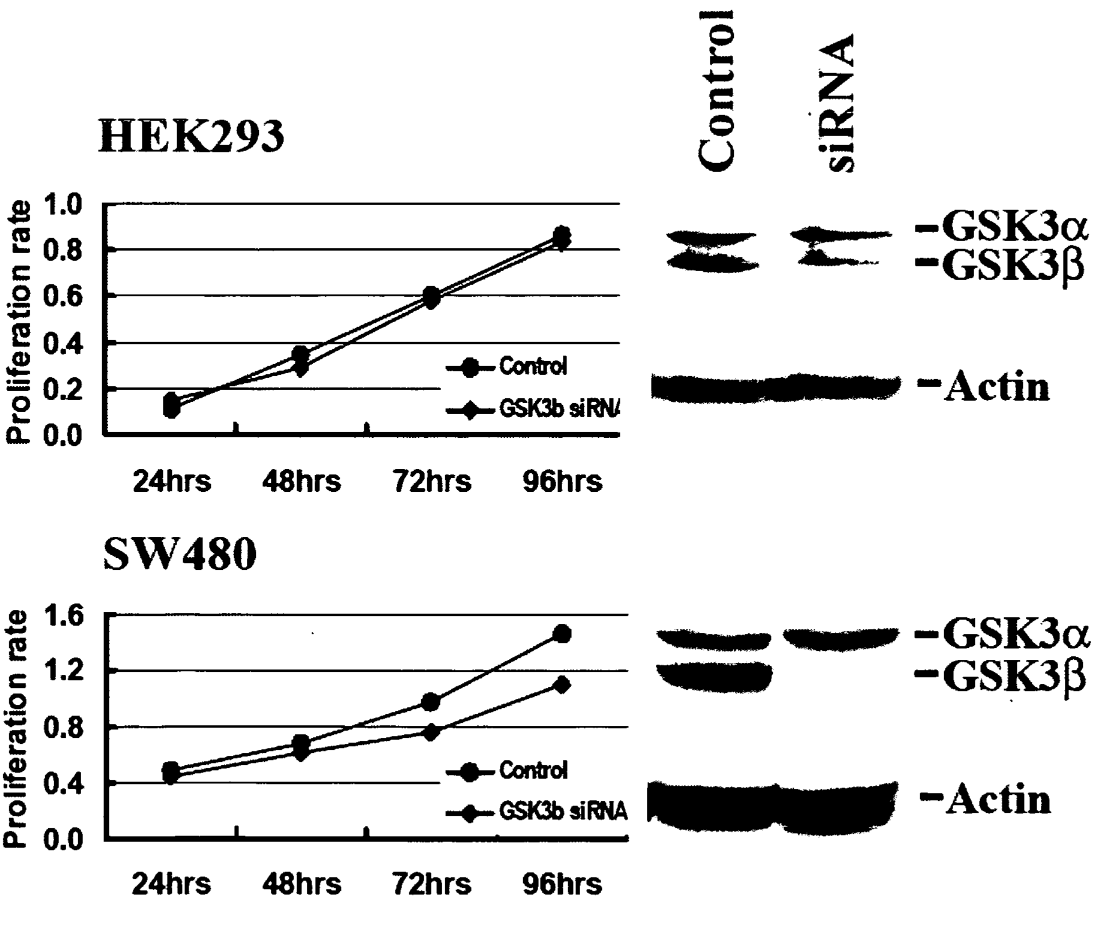
HEK293
Control
siRNA
Proliferation rate
1.0
0.8
0.6
0.4
0.2
0.0
Control
GSK3b siRNA
24hrs
48hrs
72hrs
96hrs
-GSK3α
-GSK3β
-Actin
SW480
Proliferation rate
1.6
1.2
0.8
0.4
0.0
Control
GSK3b siRNA
24hrs
48hrs
72hrs
96hrs
-GSK3α
-GSK3β
-Actin

Fig. 6

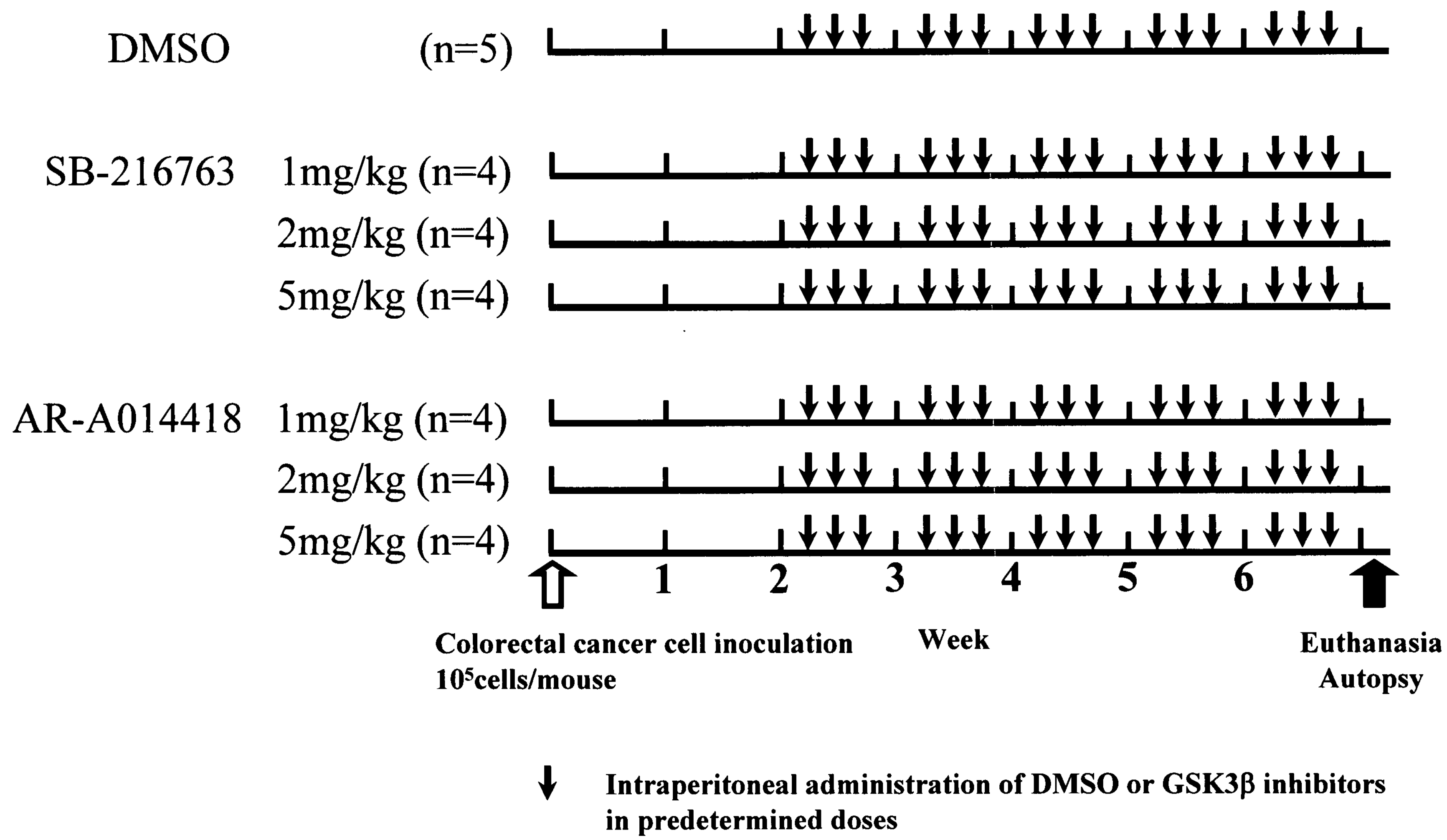
DMSO (n=5)
SB-216763 1mg/kg (n=4)
2mg/kg (n=4)
5mg/kg (n=4)
AR-A014418 1mg/kg (n=4)
2mg/kg (n=4)
5mg/kg (n=4)
1
2
3
4
5
6
Week
Colorectal cancer cell inoculation
10^5cells/mouse
Euthanasia
Autopsy
Intraperitoneal administration of DMSO or GSK3β inhibitors
in predetermined doses

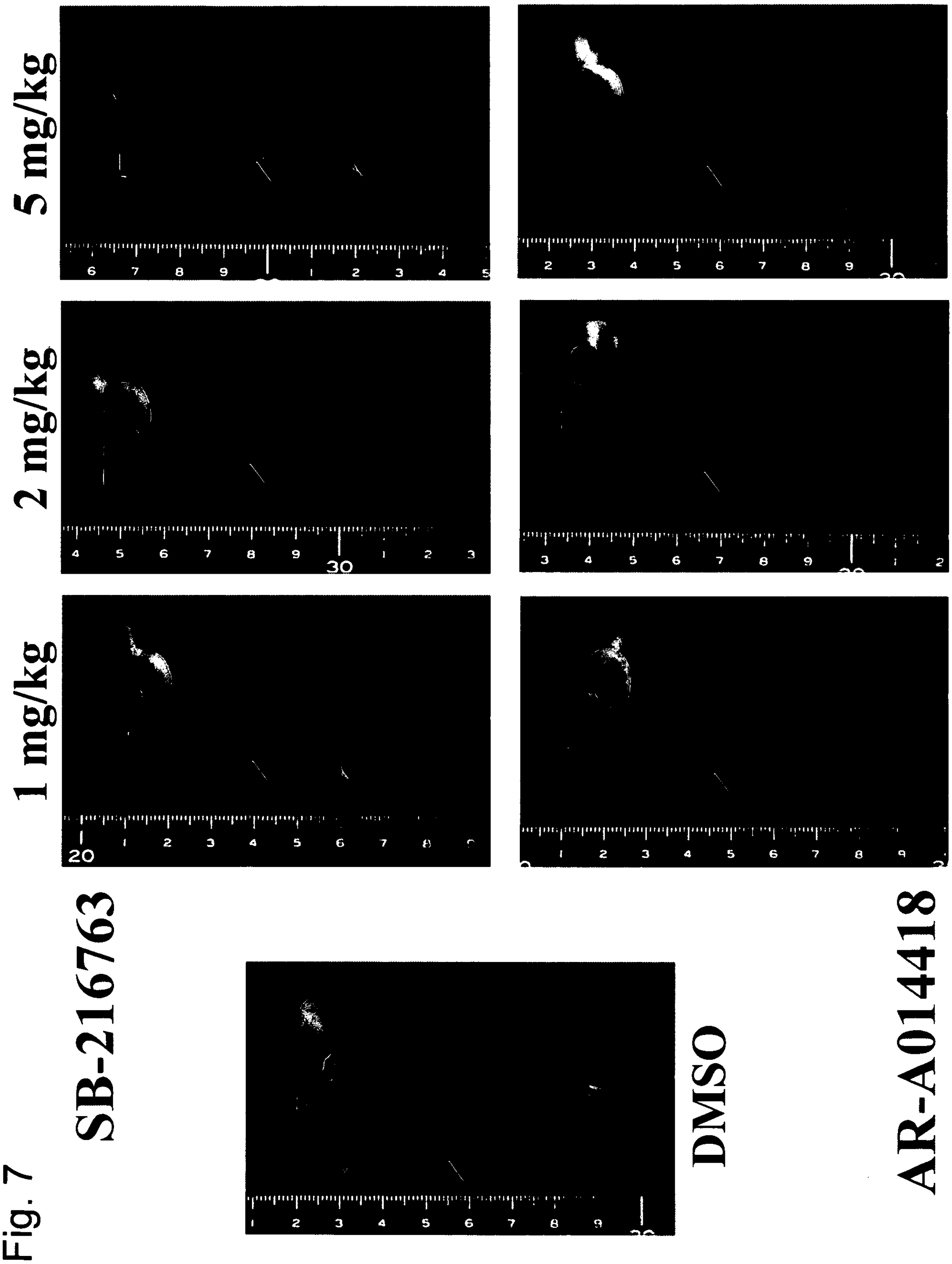


Fig. 7

Fig. 8

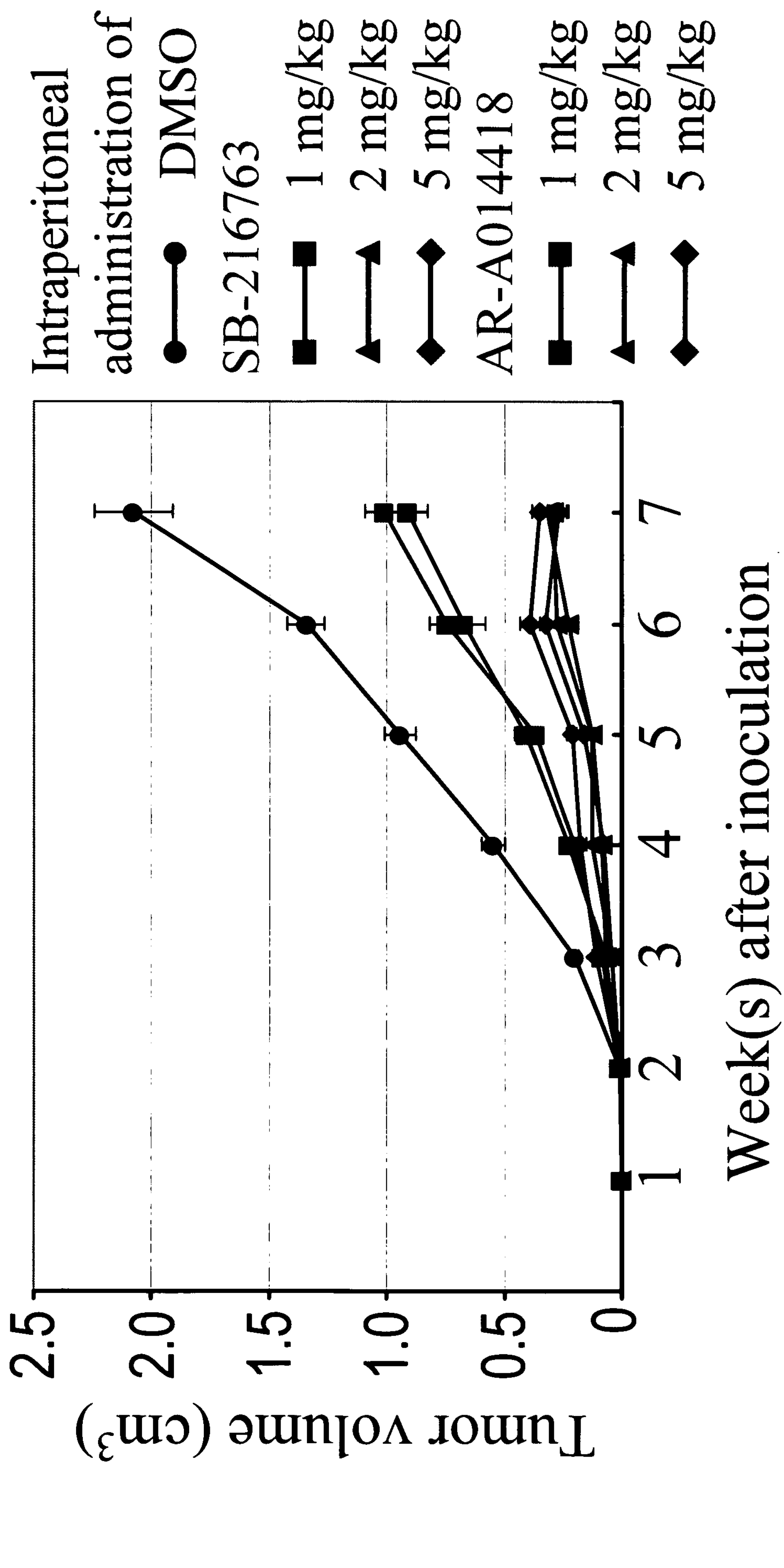
Intraperitoneal
administration of
DMSO
SB-216763
1 mg/kg
2 mg/kg
5 mg/kg
AR-A014418
1 mg/kg
2 mg/kg
5 mg/kg
Tumor volume (cm3)
2.5
2.0
1.5
1.0
0.5
0
Week(s) after inoculation
1
2
3
4
5
6
7

Fig. 9

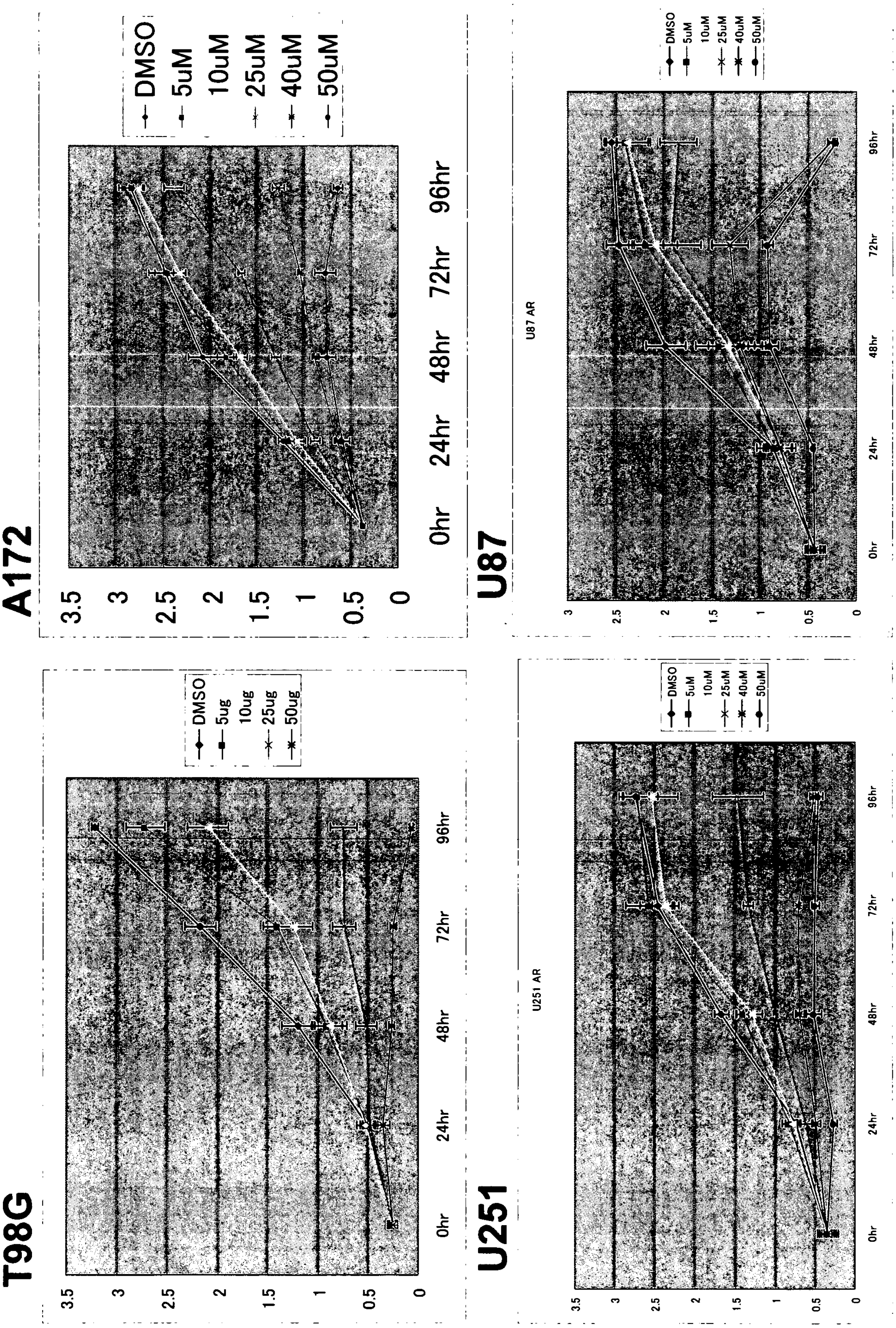
T98G
DMSO
5ug
10ug
25ug
50ug
0hr
24hr
48hr
72hr
96hr
A172
DMSO
5uM
10uM
25uM
40uM
50uM
U251
U251 AR
DMSO
5uM
10uM
25uM
40uM
50uM
U87
U87 AR
DMSO
5uM
10uM
25uM
40uM
50uM

# Fig. 10

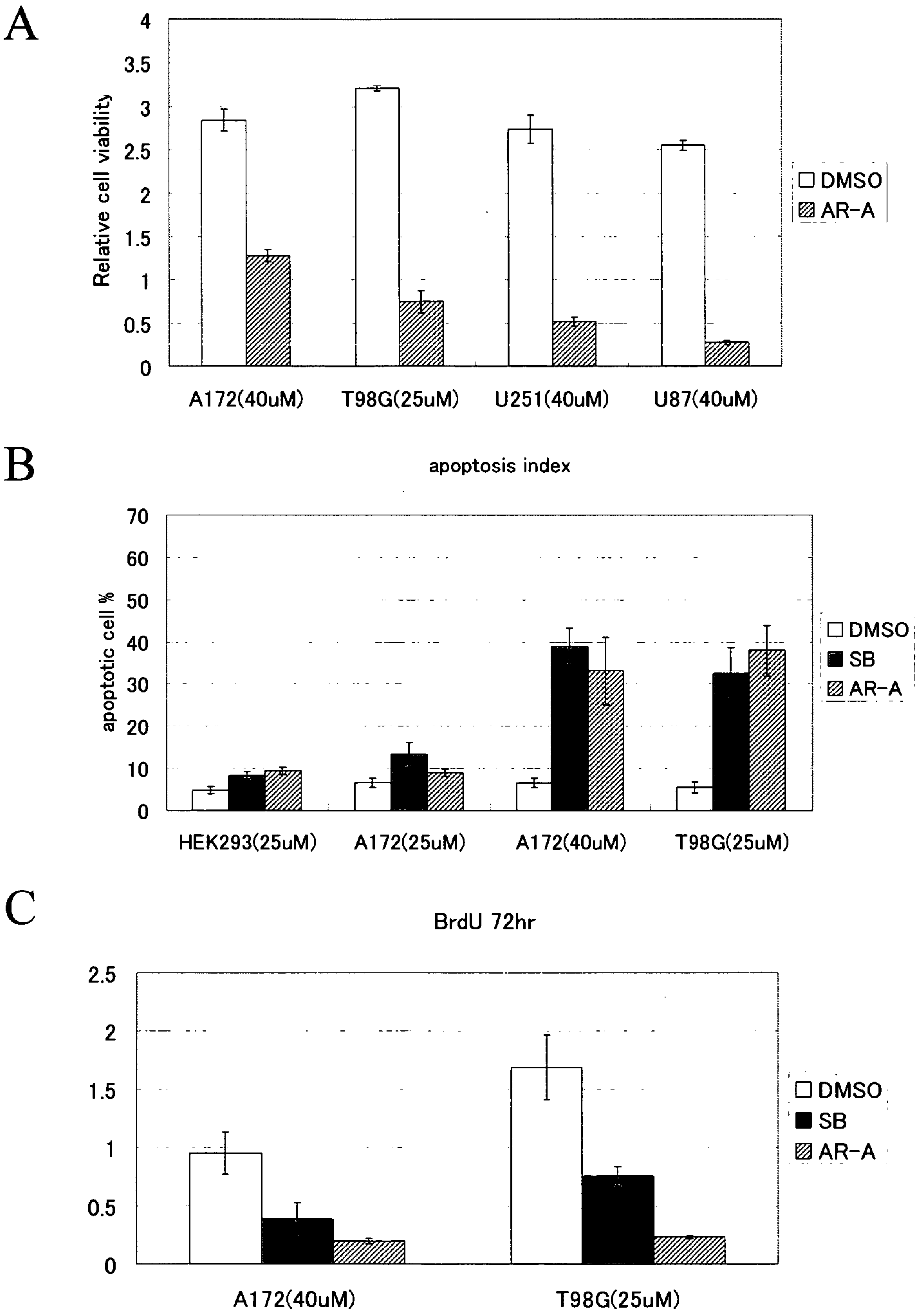
A
Relative cell viability
4
3.5
3
2.5
2
1.5
1
0.5
0
A172(40uM)
T98G(25uM)
U251(40uM)
U87(40uM)
DMSO
AR-A
B
apoptosis index
apoptotic cell %
70
60
50
40
30
20
10
0
HEK293(25uM)
A172(25uM)
A172(40uM)
T98G(25uM)
DMSO
SB
AR-A
C
BrdU 72hr
2.5
2
1.5
1
0.5
0
A172(40uM)
T98G(25uM)
DMSO
SB
AR-A

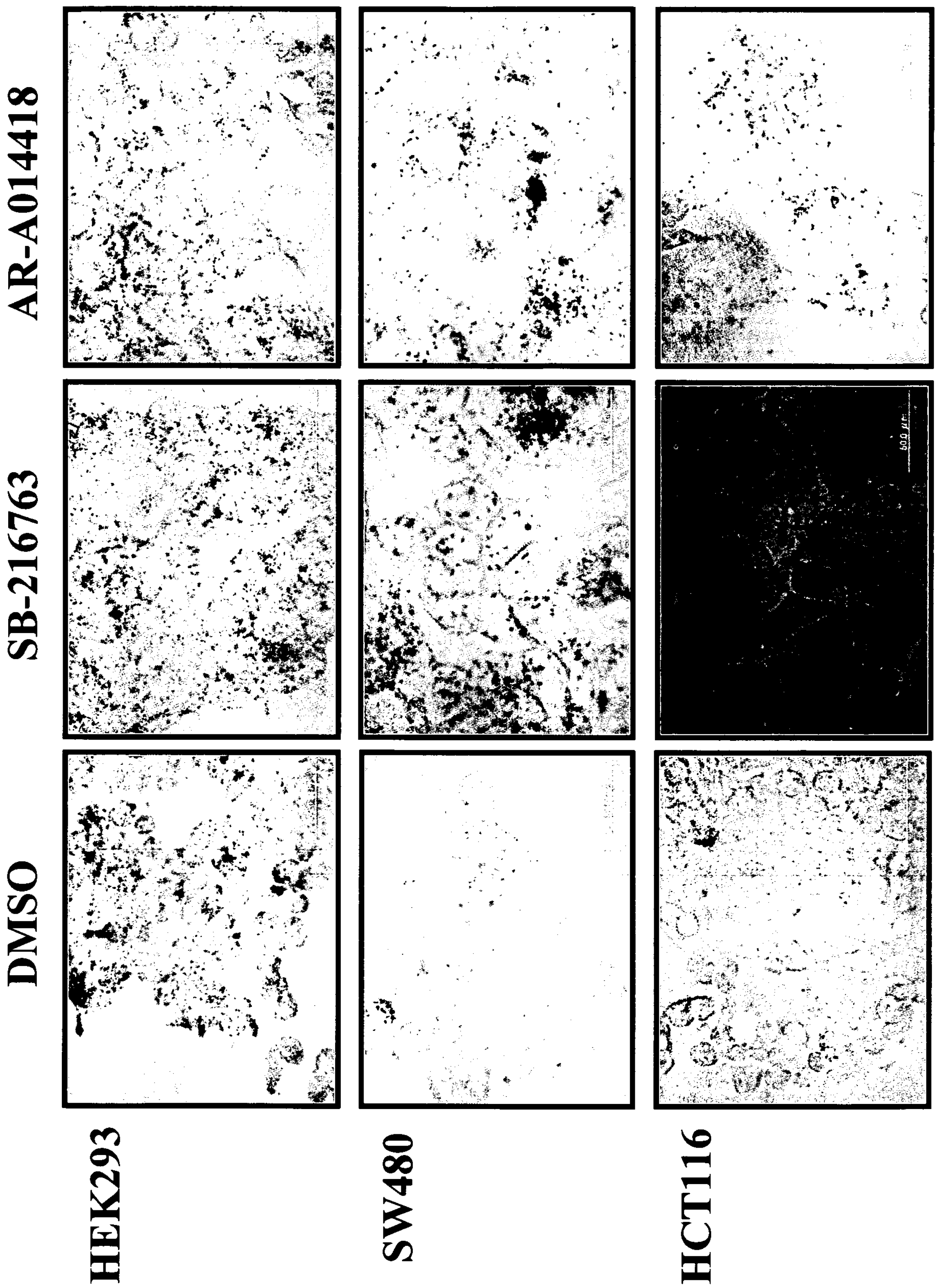


Fig. 11

Fig. 12

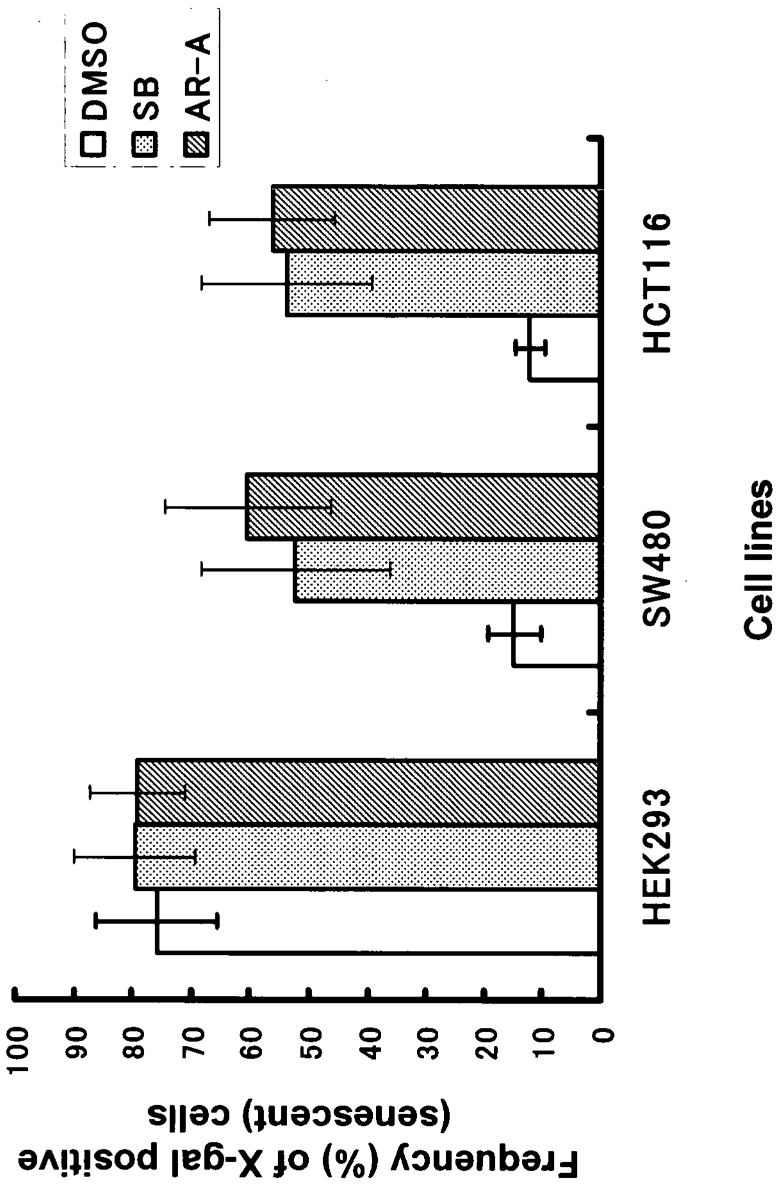
DMSO
SB
AR-A
Frequency (%) of X-gal positive (senescent) cells
0
10
20
30
40
50
60
70
80
90
100
HEK293
SW480
HCT116
Cell lines

Fig. 13

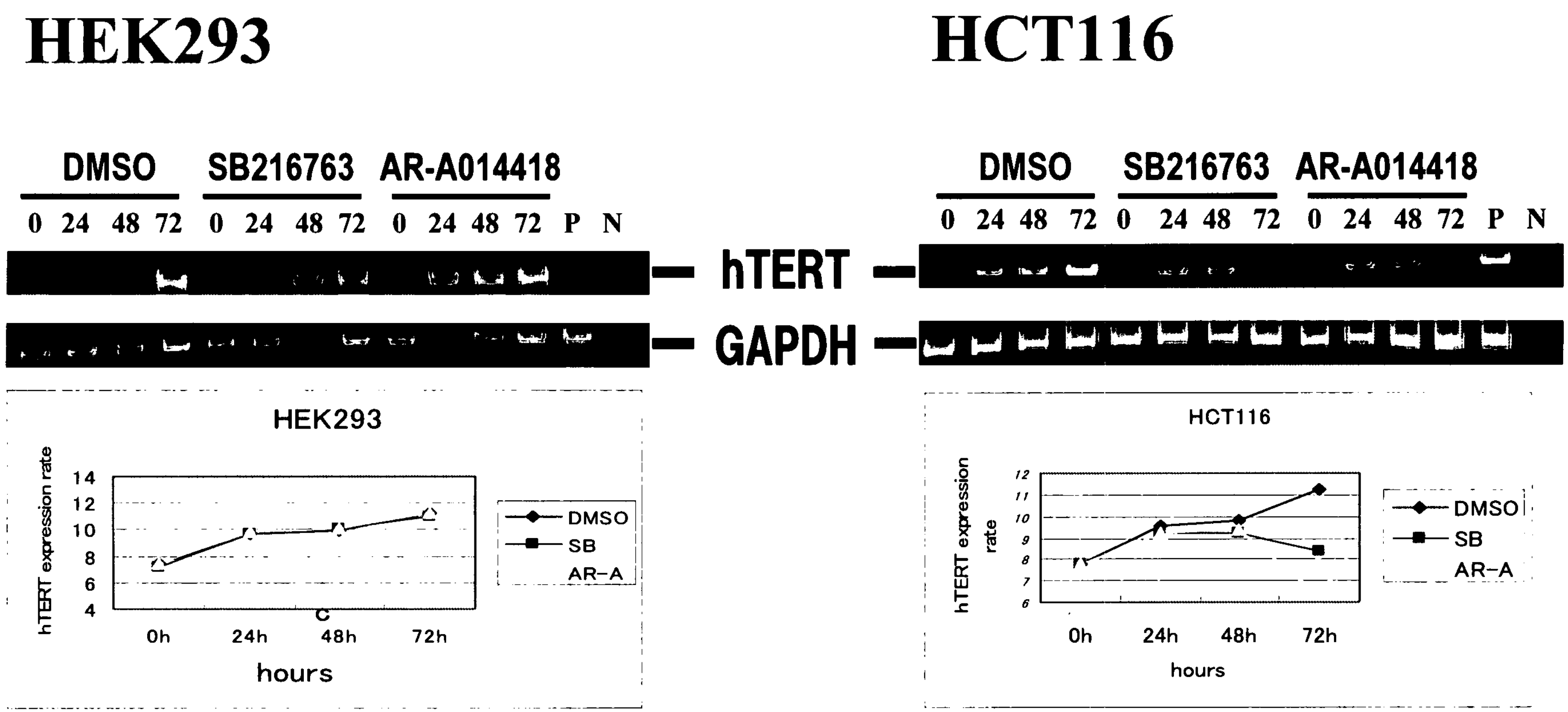


P: positive control
N: negative control

Fig. 14

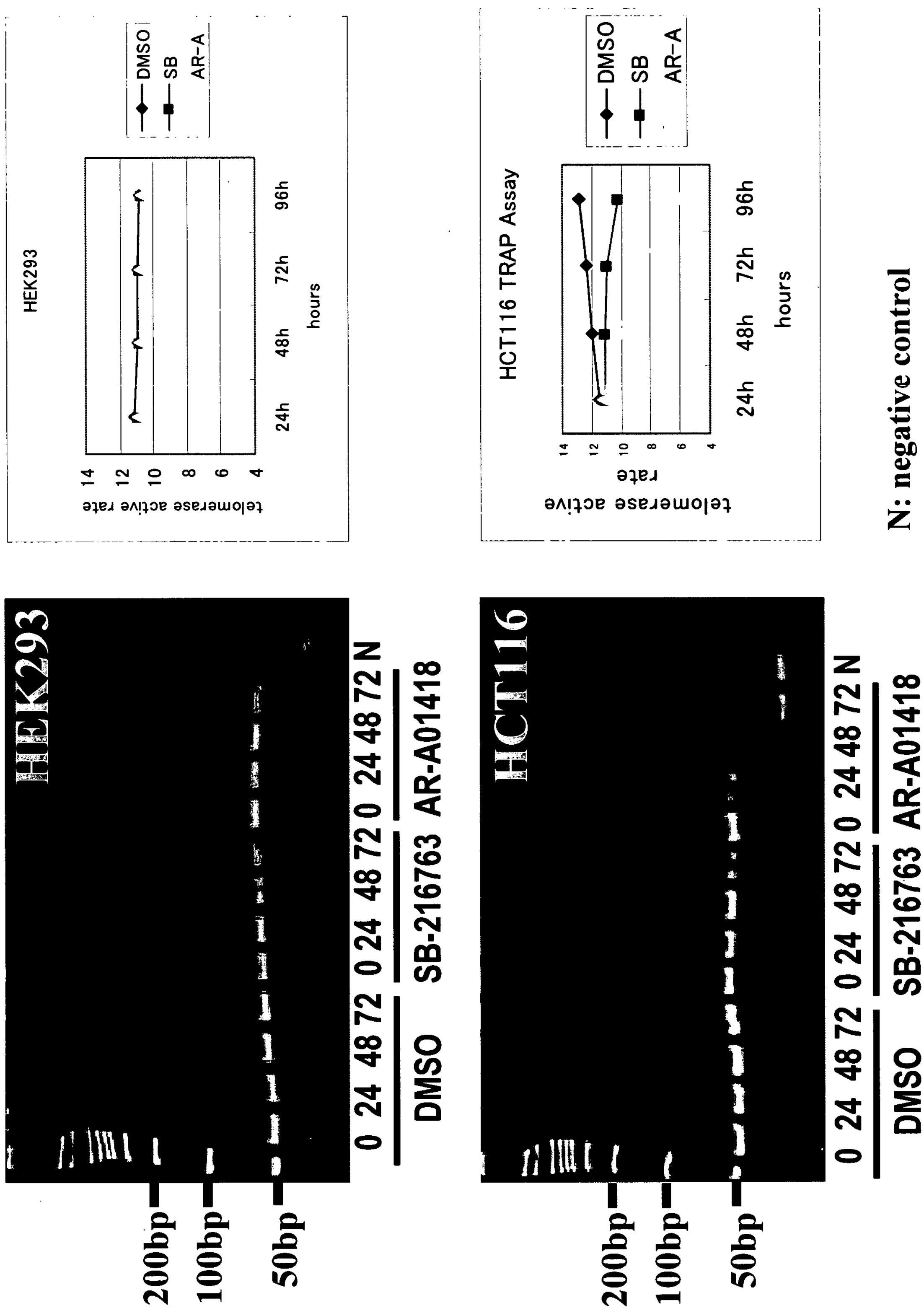
HEK293
200bp
100bp
50bp
0 24 48 72 0 24 48 72 0 24 48 72 N
DMSO
SB-216763
AR-A01418
HCT116
HEK293
telomerase active rate
14
12
10
8
6
4
24h
48h
72h
96h
hours
DMSO
SB
AR-A
HCT116 TRAP Assay
N: negative control

Fig. 15

A

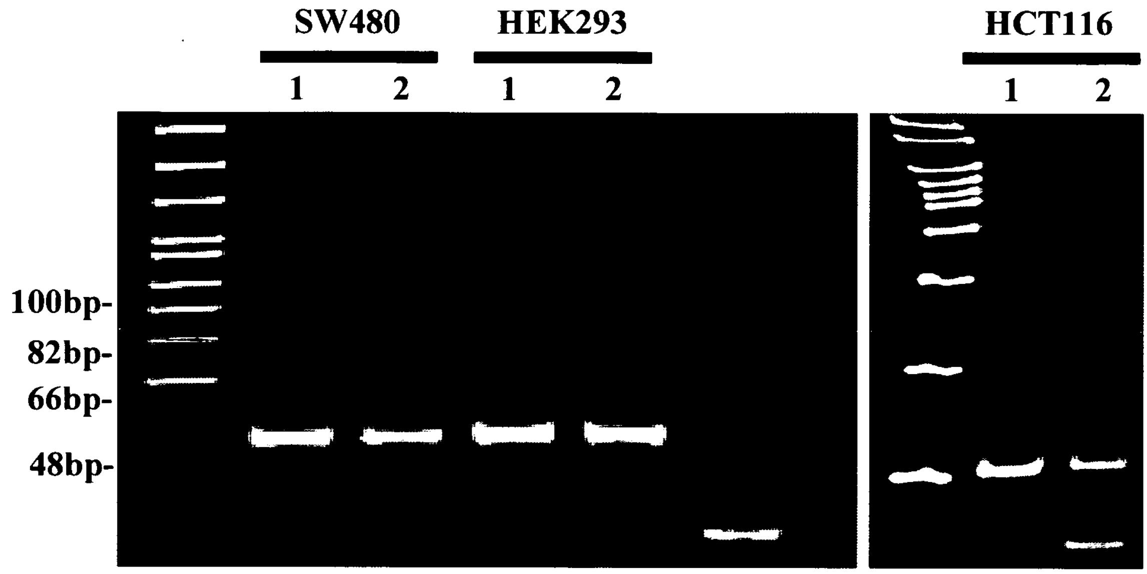


Lane1: pKD-v1 plasmid
Lane2: pKD-GSK3-v1 plasmid transfected

B

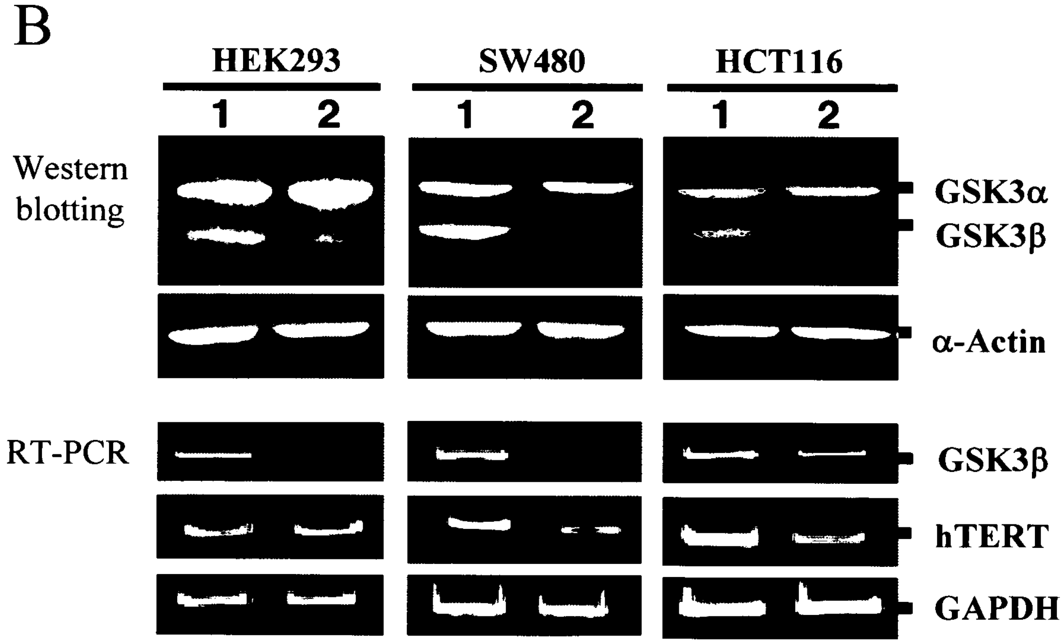


Lane1: pKD-v1 plasmid
Lane2: pKD-GSK3-v1 plasmid transfected

Fig. 16

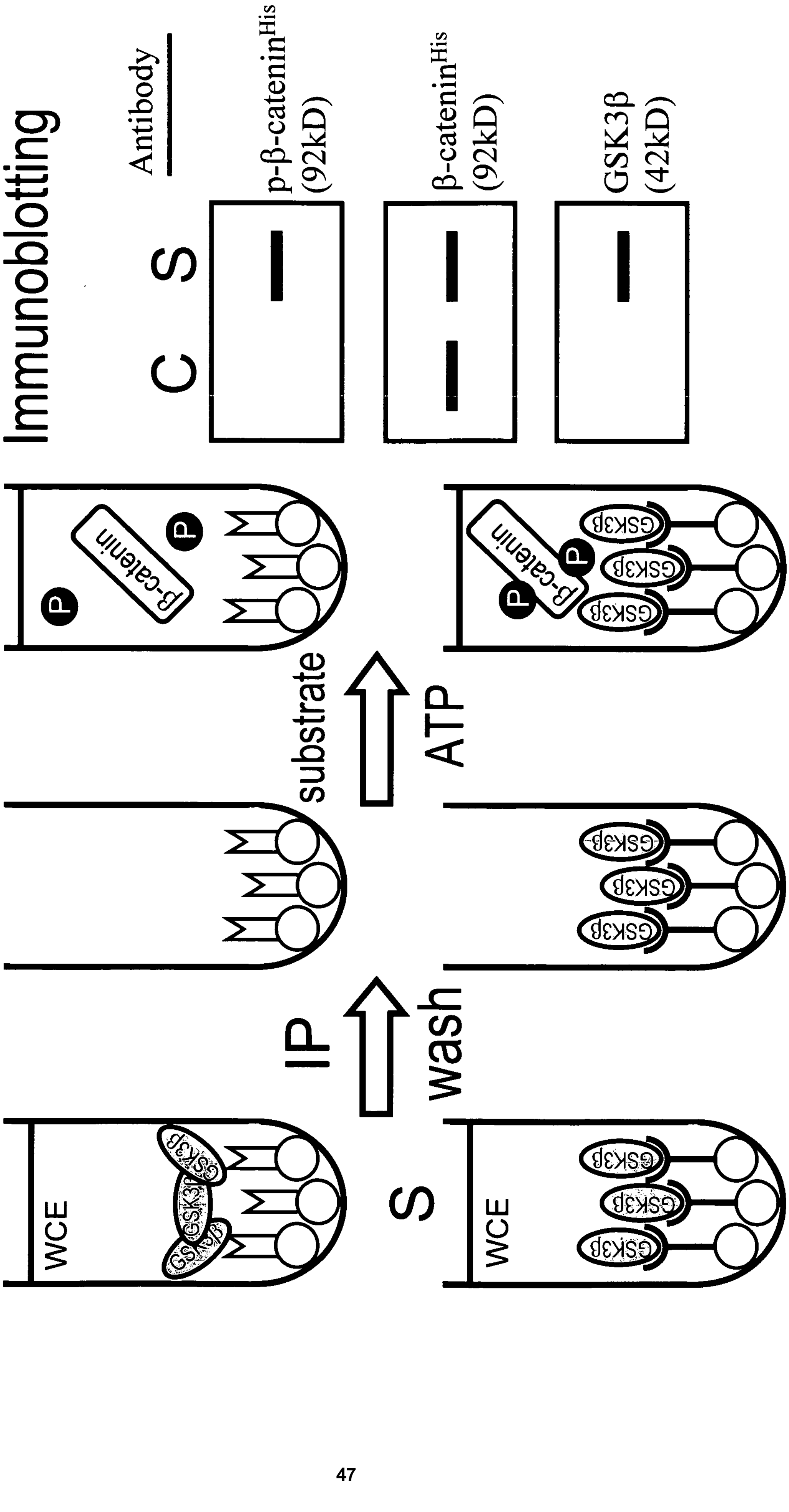
C
WCE
GSK3β
S
WCE
GSK3β
IP
wash
substrate
ATP
β-catenin
P
Immunoblotting
C S
Antibody
p-β-catenin$^{His}$
(92kD)
β-catenin$^{His}$
(92kD)
GSK3β
(42kD)

Fig. 17

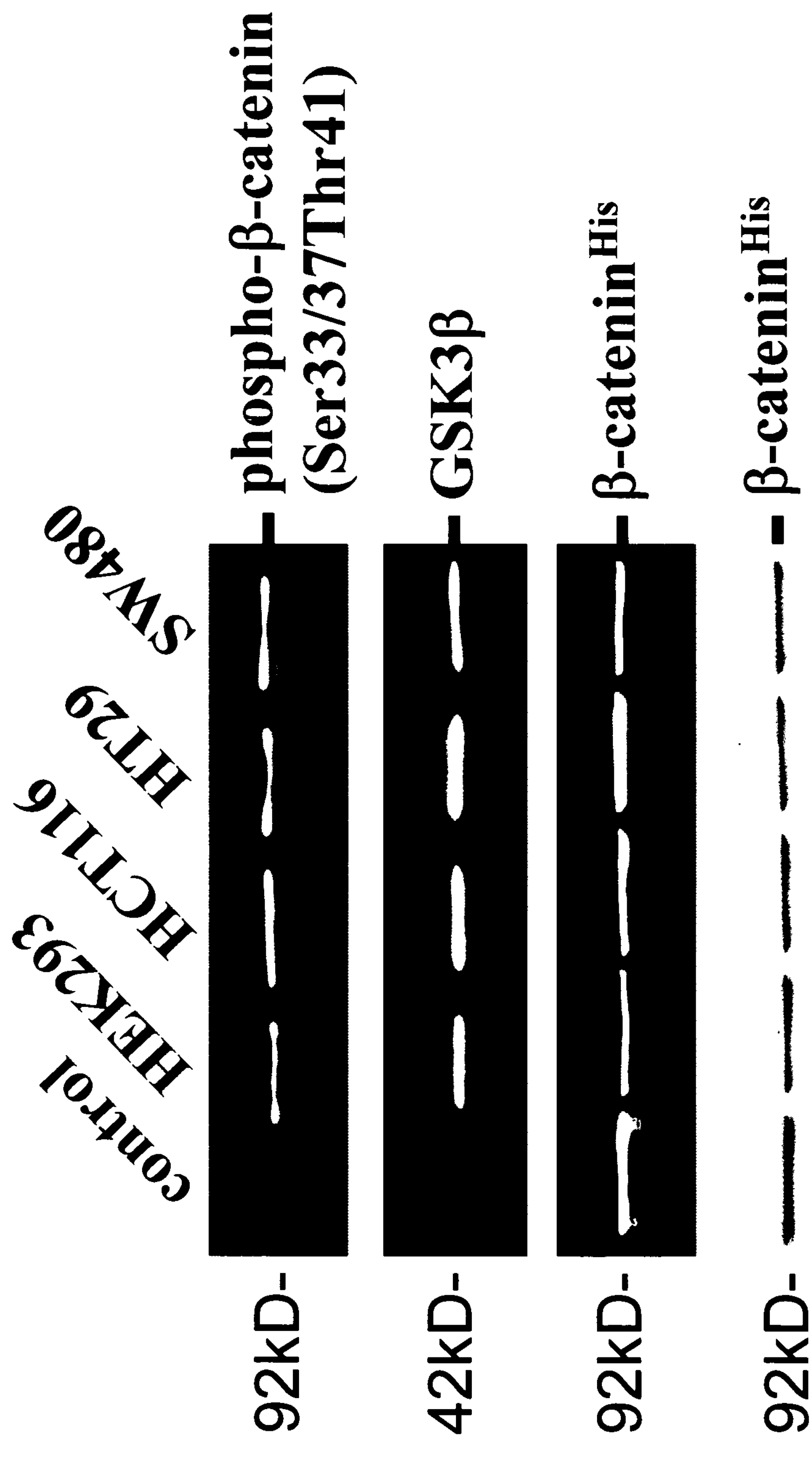


Control: immunoprecipitation with non-immune mouse IgG

Fig. 18

A

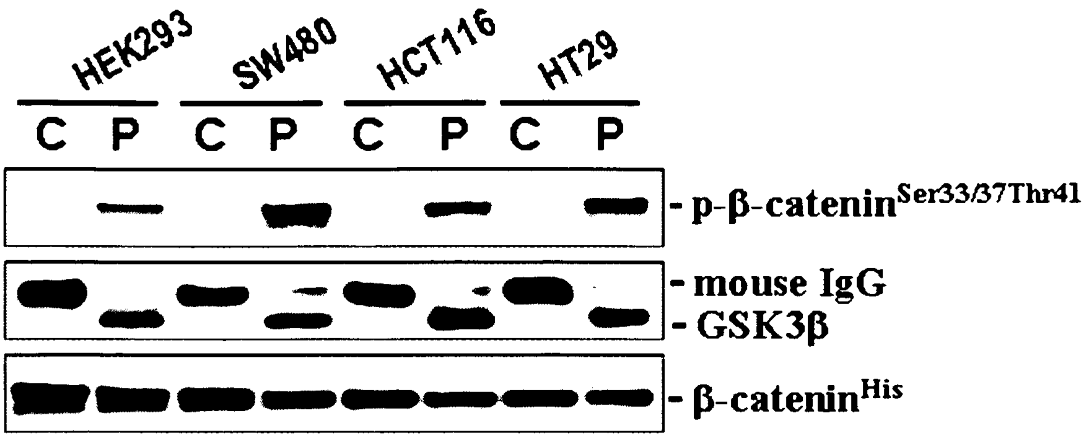
HEK293
SW480
HCT116
HT29
C P C P C P C P
- p-β-catenin Ser33/37Thr41
- mouse IgG
- GSK3β
- β-catenin His

B

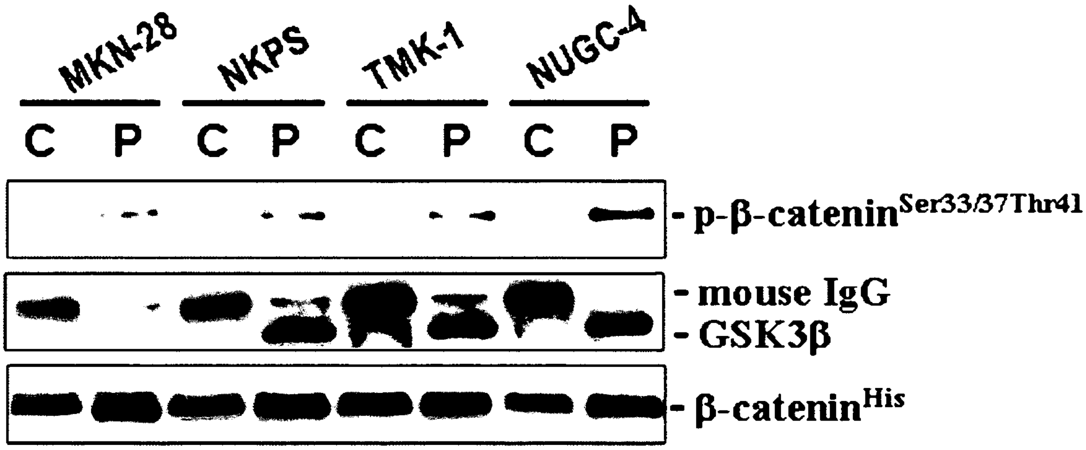
MKN-28
NKPS
TMK-1
NUGC-4
C P C P C P C P
- p-β-catenin Ser33/37Thr41
- mouse IgG
- GSK3β
- β-catenin His

C

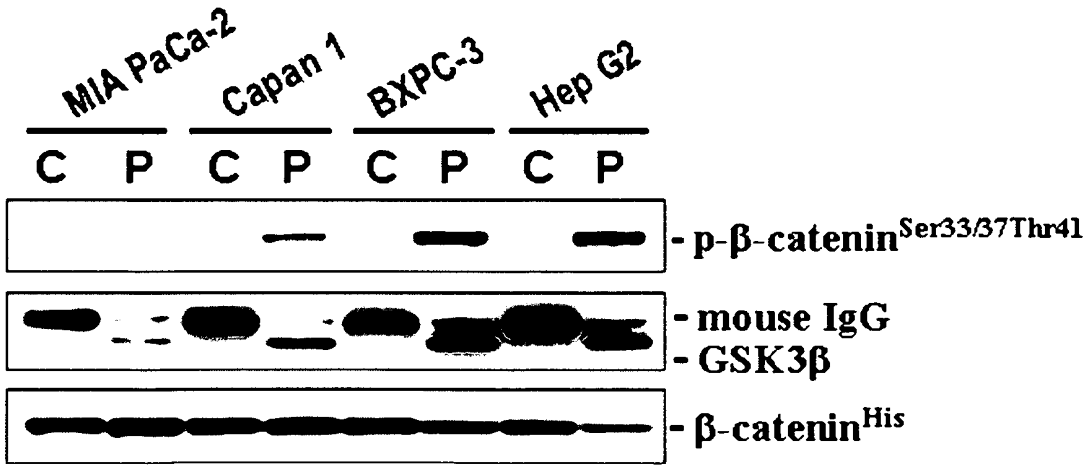
MIA PaCa-2
Capan 1
BXPC-3
Hep G2
C P C P C P C P
- p-β-catenin Ser33/37Thr41
- mouse IgG
- GSK3β
- β-catenin His

Fig. 19

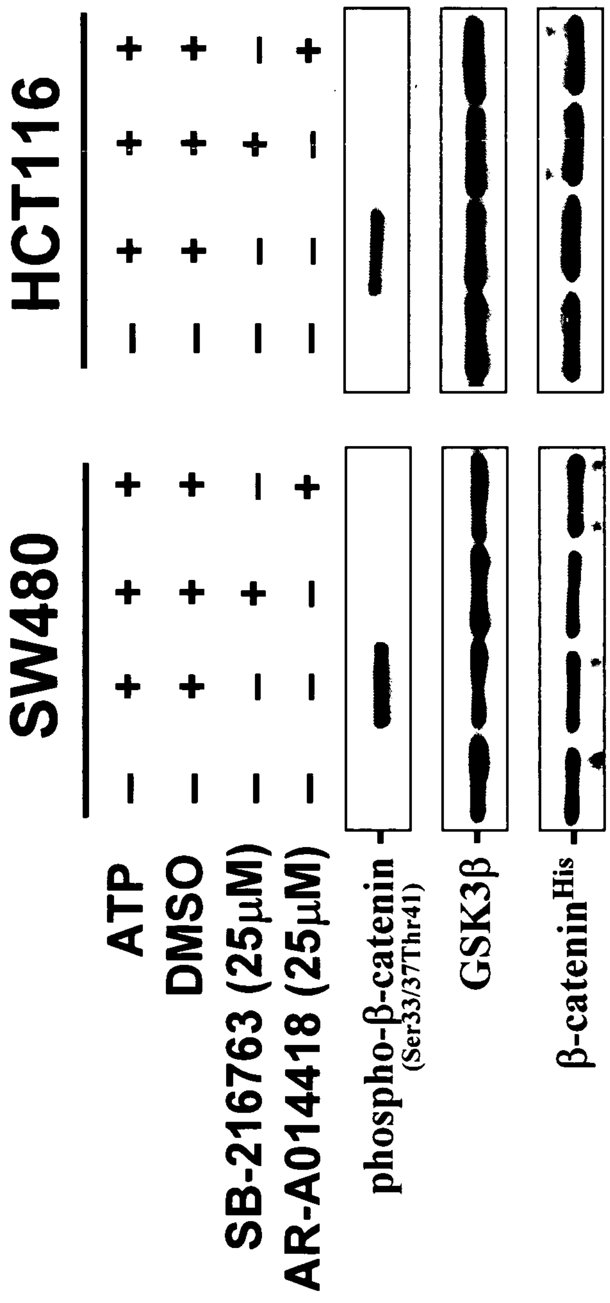
SW480
HCT116
ATP
DMSO
SB-216763 (25μM)
AR-A014418 (25μM)
phospho-β-catenin (Ser33/37Thr41)
GSK3β
β-catenin His

**INTERNATIONAL SEARCH REPORT**

International application No.
PCT/JP2006/300160

A. CLASSIFICATION OF SUBJECT MATTER
**C07D277/58**(2006.01), **A61K31/426**(2006.01), **A61P35/00**(2006.01), **C07D403/04**(2006.01), **A61K31/404**(2006.01), **C12N15/09**(2006.01)

According to International Patent Classification (IPC) or to both national classification and IPC

B. FIELDS SEARCHED

Minimum documentation searched (classification system followed by classification symbols)
C07D277/58, A61K31/426, A61P35/00, C07D403/04, A61K31/404

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
BIOSIS(STN), CAplus(STN), EMBASE(STN), MEDLINE(STN), REGISTRY(STN), WPI(DIALOG), JSTPlus(JOIS)

C. DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2002-528538 A (Pharmacia & Upjohn S.p.A.), 03 September, 2002 (03.09.02), Claims; Par. Nos. [0012], [0091] to [0092]; page 77, line 28 to page 78, line 5<br>& WO 00/26203 A1 & AU 200010447 A<br>& BR 9914868 A & NO 200102058 A<br>& EP 1124811 A1 & CZ 200101413 A3<br>& ZA 200102869 A & SK 200100475 A<br>& KR 2001085984 A & CN 1325390 A<br>& HU 200104167 A2 & MX 2001004277 A1<br>& US 2003/0187040 A1 & NZ 510967 A<br>& AU 771166 B2 & US 2004/0157827 A1<br>& AU 2004202678 A1 & US 6863647 B2 | 6,7,9,10<br>6,7,9,10<br>8 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 23 March, 2006 (23.03.06) | 11 April, 2006 (11.04.06) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2006/300160

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | JP 2001-522842 A (BRISTOL-MYERS SQUIBB CO.), 20 November, 2001 (20.11.01), Claims; Par. Nos. [0050] to [0057]; example 233<br>& WO 99/24416 A1 & AU 9912955 A<br>& US 6040321 A & NO 200002153 A<br>& ZA 9810332 A & EP 1042307 A1<br>& BR 9814124 A & AU 730607 B<br>& CN 1278806 A & CZ 200001744 A3<br>& US 6262096 B1 & KR 2001031896 A<br>& MX 2000004488 A1 & HU 200004559 A2<br>& NZ 503828 A & KR 2003036862 A<br>& RU 2211839 C2 & NO 316773 B1<br>& IL 135589 A & TW 593292 A<br>& KR 454426 B | 6,7,9,10<br>6,7,9,10<br>8 |
| Y<br>A | JP 2004-536111 A (Astra Zeneca AB.), 02 December, 2004 (02.12.04), Full text<br>& WO 03/04478 A1 & EP 1406883 A1<br>& AU 2002319988 A1 & US 2005/0054698 A1 | 6,7,9,10<br>8 |
| Y<br>A | BHAT R., et al., Structural Insights and Biological Effects of Glycogen Synthase Kinase 3-specific Inhibitor AR-A014418, THE JOURNAL OF BIOLOGICAL CHEMISRY, 2003, 278(46), pages 45937-45945, full text | 6,7,9,10<br>8,11-16 |
| Y<br>A | MEIJER L. et al., Pharmacological inhibitors of glycogen synthase kinase 3, TRENDS in Pharmacological Sciences, 2004, 25(9), pages 471-480; Table 1; Fig. 2; page 477, right column, lines 3 to 42 | 6,7,9,10<br>8,11-16 |
| X<br>Y<br>A | LIAO X., et al., Glycogen synthase kinase-3.β suppression eliminates tumor necrosis factor-related apoptosis-inducing ligand resistance in prostate cancer, Molecular Cancer Therapeutics, 2003, 2, pages 1215-1222 | 6,7<br>9,10<br>8 |
| X<br>Y<br>A | MAZOR M., et al., Inhibition of glycogen synthase kinase-3. represses androgen receptor activity and prostate cancer cell growth, Oncogene, 2004, 23, pages 7882-7892, | 6,7<br>9,10<br>8 |
| A | JP 2004-504838 A (Kairon Corp.), 19 February, 2004 (19.02.04),<br>& WO 02/10357 A2 & AU 200180812 A<br>& US 2002/082408 A1 & US 6465231 B2<br>& US 2003/0077798 A1 & EP 1319064 A2<br>& CN 1468302 A & US 6716624 B2<br>& US 2005/0048511 A1 & AU 2001280812 A8 | 11-16 |

Form PCT/ISA/210 (continuation of second sheet) (April 2005)

**INTERNATIONAL SEARCH REPORT**

International application No. PCT/JP2006/300160

C (Continuation). DOCUMENTS CONSIDERED TO BE RELEVANT

| Category* | Citation of document, with indication, where appropriate, of the relevant passages | Relevant to claim No. |
|---|---|---|
| P,X | SHAKOORI A. et al., Deregulated GSK3□ activity in colorectal cancer: Its association with tumor cell survival and proliferation, Biochemical and Biophysical Research Communications, 2005, 334, pages 1365-1373 | 6-16 |
| P,X | Toshinari HARA et al., "Daichogan ni okeru GSK3 β no Wnt Signal Hi Izonsei Kasseisei to Kino: Gan Chiryo no Aratana Bunshi Hyoteki", Dai 64 Kai The Japanese Cancer Association Sokai Kiji, 15 August, 2005 (15.08.05), pages 38 to 39; W-019 | 6,7,9,10 |
| P,X<br>P,Y | WO 2006/006939 A1 (AGENCY FOR SCIENCE, TECHNOLOGY AND RESEARCH), 19 January, 2006 (19.01.06), (Family: none) | 6-16<br>6-16 |
| P,X<br>P,Y | WO 2006/018633 A1 (IMPERIAL COLLEGE INNOVATIONS Ltd), 23 February, 2006 (23.02.23), (Family: none) | 6-16<br>6-16 |

Form PCT/ISA/210 (continuation of second sheet) (April 2005)

**INTERNATIONAL SEARCH REPORT**

International application No.
PCT/JP2006/300160

**Box No. II Observations where certain claims were found unsearchable (Continuation of item 2 of first sheet)**

This international search report has not been established in respect of certain claims under Article 17(2)(a) for the following reasons:

1. [X] Claims Nos.: 1-5
because they relate to subject matter not required to be searched by this Authority, namely:
The inventions as set forth in claims 1 to 5 pertain to methods for treatment of the human body by therapy and thus relate to a subject matter which this International Searching Authority is not required to search (Article 17(2)(a)(i) of the PCT, Rule 39.1(iv) of the Regulations under the PCT).

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
(See extra sheet.)

1. [X] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee..

[ ] The additional search fees were accompanied by the applicant's protest but the applicable protest fee was not paid within the time limit specified in the invitation.

[X] No protest accompanied the payment of additional search fees.

Form PCT/ISA/210 (continuation of first sheet (2)) (April 2005)

INTERNATIONAL SEARCH REPORT

International application No. PCT/JP2006/300160

Continuation of Box No.III of continuation of first sheet(2)

Claims 6 to 16

These claims have the following inventions.

(1) A medicinal composition for treating cancer which contains, as the active ingredient, a compound having a structure of the formula (I) or (II) or a pharmacologically acceptable salt thereof (claims 6 to 10).

(2) A method of evaluating the effect as an anticancer agent of a test substance by using, as an indication, an effect of inhibiting the activation of glycogen synthase kinase 3β (GSK3β) by the phosphorylation at the 216th tyrosine residue thereof (claims 11 to 16).

It appears that the matter common to these invention groups resides in a medicinal composition for treating cancer. However, such a matter has been publicly known by a person skilled in the art (see, for example, JP 2002-528538 A). Thus, it does not appear that there is a technical relationship between these groups of inventions involving the same or corresponding special technical feature(s) and these invention groups are not considered as being so linked as to form a single general inventive concept. Thus, the requirement of unity of invention is not fulfilled thereby.

It appears that the technical matter common to the inventions according to claims 6 to 10 relating to a medicinal composition for treating cancer which contains a compound having the structure of the formula (I) as the active ingredient and the inventions relating to a medicinal composition for treating cancer which contains a compound having the structure of the formula (II) as the active ingredient resides in a medicinal composition for treating cancer. However, medicinal compositions for treating cancer have been known in public and no common structure can be found out between the compounds represented by the formulae (I) and (II). Thus, it does not appear that there is a technical relationship concerning the above-described technical matter involving the same or corresponding special technical feature(s) and these invention groups are not considered as being so linked as to form a single general inventive concept.

Such being the case, it is recognized that the present case has the following three invention groups that do not mutually fulfill the requirement of unity of invention.

a) The inventions according to claims 6 to 10 comprising a compound having the structure of the formula (I) as the active ingredient.

b) The inventions according to claims 6 to 10 comprising a compound having the structure of the formula (II) as the active ingredient.

c) The inventions according to claims 11 to 16.

Form PCT/ISA/210 (extra sheet) (April 2005)

INTERNATIONAL SEARCH REPORT

International application No.
PCT/JP2006/300160

Claims 6 and 8 to 10

In the structural formula presented as the formula (I) in claim 6, it is stated that the substituent $Q_1$ is "-$(CH_2)_n$-Ar-O- (wherein n is an integer of from 1 to 5; and Ar is substituted or unsubstituted aryl)".

However, it is unclear what the terminal substituent having the structure "-O-" is and to which position of Ar it is attached. Thus, the invention according to this claim cannot be clearly understood merely based on such statement. In the description, N-(4-methoxybenzyl)-N'-(5-nitro-1,3-thiazol-2-yl)urea alone is presented and no other case was confirmed in practice.

Accordingly, the inventions according to claims 6 and 8 to 10 are unclear based on the statements in these claims and the description and these inventions are not fully supported by the description (PCT Articles 5 and 6).

Such being the case, prior art was searched by considering the moiety "-$(CH_2)_n$-Ar-O-" as ""-$(CH_2)_n$-Ar- (wherein n is an integer of from 1 to 5; and Ar is an aryl group optionally having a substituent)" in this report.

(Individual substituents other than the above-described one have the same meanings as defined in the description.)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2004528837 A
- JP 2004504838 A
- JP 2004507545 A
- JP 2005000133 A
- JP 2005536517 A
- JP 2005535593 A
- JP 2005534713 A
- JP 2005533066 A
- JP 2005531609 A
- JP 2005526835 A
- JP 2005526814 A
- JP 2005519087 A
- JP 2005519086 A
- JP 2005516961 A
- JP 2005516960 A
- JP 2005516948 A
- JP 2005513082 A
- JP 2005509641 A
- JP 2005505515 A
- JP 2005504810 A
- JP 2005504809 A
- JP 2005501800 A
- JP 2004053831 A
- JP 2004536111 A
- JP 2004536110 A
- JP 2004527486 A
- JP 2004522777 A
- JP 2004516297 A
- JP 2004515499 A
- JP 2004505078 A
- JP 2003528095 A
- JP 2003527303 A
- JP 2003516974 A
- JP 2002527419 A
- JP 1081345 A
- JP H01233281 A
- AR 014418 A
- WO 2003004478 A
- EP 328026 A
- JP 1233281 A


### Non-patent literature cited in the description


- **JASS JR ; WHITEHALL VLJ ; YOUNG J ; LEGGETT BA.** Emerging concepts in colorectal neoplasia. *Gastroenterology,* 2002, vol. 123 (3), 862-76
- **WONG NA ; PIGNATELLI M.** β-catenin-a linchpin in colorectal carcinogenesis. *Am J Pathol,* 2002, vol. 160 (2), 389-401
- **POLAKIS P.** The oncogenic activation of beta-catenin. *Curr Opin Genet Dev,* 1999, vol. 9 (1), 15-21
- **OUGOLKOV AV ; YAMASHITA K ; MAI M ; MINAMOTO T.** Oncogenic β-catenin and MMP-7 (matrilysin) cosegregate in late-stage clinical colon cancer. *Gastroenterology,* 2002, vol. 122 (1), 60-71
- **ZHANG B ; OUGOLKOV A ; YAMASHITA K ; TAKAHASHI Y ; MAI M ; MINAMOTO T.** β-catenin and ras oncogenes detect most human colorectal cancers. *Clin Cancer Res,* 2003, vol. 9 (8), 3073-9
- **MANOUKIAN AS ; WOODGETT JR.** Role of glycogen synthase kinase-3 in cancer: regulation by Wnts and other signaling pathways. *Adv Cancer Res,* 2002, vol. 84, 203-29
- **DOBLE BW ; WOODGETT JR.** GSK-3: tricks of the trade for a multi-tasking kinase. *J Cell Sci,* 2003, vol. 116, 1175-86
- **HARWOOD AJ.** Regulation of GSK-3, a cellular multiprocessor. *Cell,* 2001, vol. 105 (7), 821-4
- **KIM L ; HARWOOD A ; KIMMEL AR.** Receptor-dependent and tyrosine phosphatase-mediated inhibition of GSK3 regulates cell fate choice. *Dev Cell,* 2002, vol. 3 (4), 523-32
- **MORTON S ; DAVIS RJ ; MCLAREN A ; COHEN P.** A reinvestigation of the multiple phosphorylation of the transcription factor c-Jun. *EMBO J,* 2003, vol. 22 (15), 3876-86
- **GREGORY MA ; QI Y ; HANN SR.** Phosphorylation by glycogen synthase kinase-3 controls c-myc proteolysis and subnuclear localization. *J Biol Chem,* 2003, vol. 278 (51), 51606-12

- **PRICE MA ; KALDERON D.** Proteolysis of the Hedgehog signaling effector Cubitus interruptus requires phosphorylation by glycogen synthase kinase 3 and casein kinase 1. *Cell,* 2002, vol. 108 (6), 823-35
- **JIA J ; AMANAI K ; WANG G ; TANG J ; WANG B ; JIANG J.** Shaggy/GSK3 antagonizes Hedgehog signalling by regulating cubitus interruptus. *Nature,* 2002, vol. 416 (6880), 548-52
- **HOEFLICH KP ; LUO J ; RUBIE EA ; TSAO MS ; JIN O ; WOODGETT JR.** Requirement for glycogen synthase kinase-3β in cell survival and NF-κB activation. *Nature,* 2000, vol. 406 (6791), 86-90
- **SCHWABE RF ; BRENNER DA.** Role of glycogen synthase kinase-3 in TNF-α-induced NF-κB activation and apoptosis in hepatocytes. *Am J Physiol Gastrointest Liver Physiol,* 2002, vol. 283 (1), G204-11
- **COHEN P ; GOEDERT M.** GSK3 inhibitors: development and therapeutic potential. *Nat Rev Drug Discov,* 2004, vol. 3 (6), 479-87
- **LIAO X ; ZHANG L ; THRASHER JB ; DU J ; LI B.** Glycogen synthase kinase-3β suppression eliminates tumor necrosis factor-related apoptosis-inducing ligand resistance in prostate cancer. *Mol Cancer Ther,* 2003, vol. 2 (11), 1215-1222
- **WANG L ; LIN H-K ; HU Y-C ; XIE S ; YANG L ; CHANG C.** Suppression of androgen receptor-mediated transactivation and cell growth by the glycogen synthase kinase 3β in prostate cells. *J Biol Chem,* 2004, vol. 279 (31), 32444-32452
- **MAZOR M ; KAWANO Y ; ZHU H ; WAXMAN J ; KYPTA RM.** Inhibition of glycogen synthase kinase-3 represses androgen receptor activity and prostate cancer cell. *Oncogene,* 2004, vol. 23 (47), 7882-7892
- **ADLER V et al.** *Proc Natl Acad Sci U.S.A.,* 1997, vol. 94, 1686-91
- **MEIJER L ; FLAJOLET M ; GREENGARD P.** *Trends in Pharmacol Sci,* 2004, vol. 25 (9), 471-480
- **MEIJER L ; FLAJOLET M ; GREENGARD P.** *Trends Pharmacol Sci,* 2004, vol. 25 (9), 471-480
- **GUPTA RA et al.** Activation of nuclear hormone receptor peroxisome proliferator-activated receptor-δ accelerates intestinal adenoma growth. *Nat Med,* 2004, vol. 10 (3), 245-247
- **LIPSHUTZ RJ et al.** *Nat Genet,* 1999, vol. 21, 20-24
- **SIVE HL ; JOHN T. ST.** *Nucleic Acids Res,* 1988, vol. 16, 10937
- **WANG Z ; BROWN DD.** *Proc Natl Acad Sci U.S.A.,* 1991, vol. 88, 11505-11509
- **LIANG P ; PARDEE AB.** *Science,* 1992, vol. 257, 967-971
- **LIANG P ; AVERBOUKH L ; KEYOMARSI K ; SAGER R ; PARDEE AB.** *Cancer Res,* 1992, vol. 52, 6966-6968
- **JOHN T. ST ; DAVIS RW.** *Cell,* 1979, vol. 16, 443-452
- **MANIATIS, T ; FRITSCH, E.F ; SAMBROOK, J.** Molecular Cloning, A Laboratory Manual. Cold Spring Harbor Laboratory Press, 1982
- **KOHLER ; MILSTEIN.** *Nature,* 1975, vol. 256, 495-497
- Monoclonal Antibody. Prenum Press, 1980, 365-367
- **SHAKOORI A et al.** *Biochem Biophys Res Commun,* 2003, vol. 312 (3), 850-7
- **ZHANG B et al.** *Clin Cancer Res,* 2003, vol. 9 (8), 3073-9
- **OUGOLKOV AV et al.** *Gastroenterology,* 2002, vol. 122 (1), 60-71
- **WONG NA et al.** *Am J Pathol,* 2002, vol. 160 (2), 389-401
- **POLAKIS P.** *Curr Opin Genet Dev,* 1999, vol. 9 (1), 15-21
- **SPIEGELMAN VS et al.** *Mol Cell,* 2000, vol. 5 (5), 877-82
- **OUGOLKOV A et al.** *J Natl Cancer Inst,* 2004, vol. 96 (15), 1161-70
- **ADJEI AA.** *J Natl Cancer Inst,* 2001, vol. 93 (14), 1062-74
- **MINAMOTO T et al.** *Expert Rev Mol Diag,* 2002, vol. 2 (6), 565-75
- **WANG L et al.** *J Biol Chem,* 2004, vol. 279 (31), 32444-52

SEQUENCE LISTING

<110> National University Corporation Kanazawa University

<120> Inhibition of cancer and method for evaluating anti-cancer drug based on GSK3beta inhibition

<130> PH-2685-PCT

<140>
<141>

<150> JP2005-133
<151> 2005-01-04

<160> 4

<170> PatentIn Ver. 2.1

<210> 1
<211> 21
<212> DNA
<213> Artificial Sequence

<220>
<223> Inventor: Minamoto, Toshinari

<220>
<223> Description of Artificial Sequence:primer

<400> 1
attcgcgaag agagtgatca t 21

<210> 2

<211> 21
<212> DNA
<213> Artificial Sequence

<220>
<223> Description of Artificial Sequence:primer

<400> 2
cactgctaac tttcatgctg c 21

<210> 3
<211> 20
<212> DNA
<213> Artificial Sequence

<220>
<223> Description of Artificial Sequence:primer

<400> 3
cggaagagtg tctggagcaa 20

<210> 4
<211> 19
<212> DNA
<213> Artificial Sequence

<220>
<223> Description of Artificial Sequence:primer

<400> 4
ggatgaagcg gagtctgga 19